# EUROPEAN PATENT SPECIFICATION

(11) **EP 4 141 420 B1**
(45) Date of publication and mention of the grant of the patent: **25.06.2025**
(21) Application number: 21929621.7
(22) Date of filing: 12.03.2021
(51) Int. Cl.: B01L 3/00, B01L 7/00, G01N 21/64, C12M 1/34

(54) **ARRAY SUBSTRATE, MICROFLUIDIC DEVICE, MICROFLUIDIC SYSTEM, AND FLUORESCENCE DETECTION METHOD**
ARRAYSUBSTRAT, MIKROFLUIDISCHE VORRICHTUNG, MIKROFLUIDISCHES SYSTEM UND FLUORESZENZERKENNUNGSVERFAHREN
SUBSTRAT À MATRICE, DISPOSITIF MICROFLUIDIQUE, SYSTÈME MICROFLUIDIQUE ET PROCÉDÉ DE DÉTECTION DE FLUORESCENCE

(43) Date of publication of application: 01.03.2023
(73) Proprietor: BOE Technology Group Co., Ltd., Beijing 100015 (CN)
(72) Inventor: DENG, Rui jun, Beijing 100176 (CN); YANG, Fan, Beijing 100176 (CN); LIU, Zhukai, Beijing 100176 (CN); DING, Ding, Beijing 100176 (CN)
(74) Representative: Studio Torta S.p.A.
(86) International application number: PCT/CN2021/080444
(87) International publication number: WO 2022/188146

(56) References cited:
- EP-A1- 3 912 720
- EP-A1- 3 912 720
- WO-A1-2020/147203
- WO-A1-2020/147203
- WO-A2-2005/028629
- CN-A- 102 168 011
- CN-A- 110 998 324
- US-A1- 2004 168 916
- US-A1- 2008 318 243
- US-A1- 2013 099 143
- US-A1- 2013 313 417

## Description

### TECHNICAL FIELD

The present disclosure relates to the field of biomedical detection, and in particular to an array substrate, a microfluidic device comprising the array substrate, a microfluidic system comprising the microfluidic device, and a fluorescence detection method.

### BACKGROUND

Polymerase Chain Reaction (PCR) is a molecular biology technique used to amplify specific deoxyribonucleic acid (DNA) fragments, which can replicate a small amount of DNA in large quantities and increase its number significantly. Digital polymerase chain reaction (dPCR) technology is a quantitative analysis technology developed on the basis of PCR that can provide digital DNA quantitative information, and greatly improves the sensitivity and accuracy in combination with the microfluidic technology. In the dPCR technology, the nucleic acid sample is sufficiently diluted so that the number of target molecules (i.e., DNA template) in each reaction unit is less than or equal to one. In each reaction unit, the target molecule is amplified through PCR, and after the amplification is completed, the fluorescent signal of each reaction unit is statistically analyzed, so as to realize the absolute quantitative detection of single-molecule DNA. Because dPCR has the advantages of high sensitivity, strong specificity, high detection throughput, accurate quantification, etc., it is widely used in various fields such as clinical diagnosis, gene instability analysis, single-cell gene expression, environmental microbial detection and prenatal diagnosis.

WO 2005/028629 A2 provides methods for simultaneously determining the genetic expression profile an individual member of a species relative to a standard genome for said species, comprising distributing a sample comprising substantially all the genetic material of said member into an array of reaction chambers on a substrate, wherein each chamber has a volume of less than about 1 microliter, and each chamber comprises a primer for a polynucleotide target within said standard genome, and a probe associated with said primer which emits a concentration dependent signal if the primer binds with said target, and the array comprises at least one chamber comprising a primer for each of the polynucleotide target within said standard genome; performing an amplification reaction on the distributed sample in the array so as to increase the concentration of polynucleotides in each of the chambers in which the polynucleotide binds to a primer; and identifying which of the reaction chambers contains a polynucleotide that has been bound to a primer, by detecting the presence of the probe associated with the primer. The reaction chambers may be formed by a microplate assembly comprising a cover and a substrate having a through-hole plate and backing sheet adhered thereto. Inherently opaque substrate materials may be used, which are formed by adding dyes to the substrate that absorb emitted fluorescence, by adding other light absorbing coatings or entities to the substrate, and by combinations thereof, thereby reducing optical crosstalk between wells of fluorescent emission signals. A "surface tension array" may be formed comprising a pattern of hydrophilic and hydrophobic areas having a plurality of hydrophilic sites, forming reaction spots (chambers), against a hydrophobic matrix, the hydrophilic sites being spatially segregated by hydrophobic regions.

CN 110 998 324 A provides a method and device for partitioning a fluidic sample. The device contains a plate containing micro-wells. The method comprises depositing a sample on one or both of the plates when the plates are in an open configuration, wherein the deposition is in the form of a single or multiple droplet of the sample, wherein at least 1/5 of the droplets has a volume that occupies more than two micro-wells and closing the plates to the closed configuration to partition the sample in the micro-wells.

US 2013/099143 A1 provides a structure for particle immobilization. The structure has a plurality of holding holes for holding respective test particles in order to detect light emitted from a substance which indicates the presence of a component for constructing each of the test particles, and the structure for particle immobilization comprises a flat plate substrate and a holding unit which is arranged on the substrate and which is formed with the plurality of holding holes. In this configuration, a light shielding film which reduces light noise is provided for the substrate or the holding unit in order that the light noise such as the background noise and the crosstalk noise can be reduced, and a large number of test particles can be optically observed highly sensitively and highly accurately.

WO 2020/147203 A1 discloses a detection chip and a use method therefor, and a reaction system. The detection chip comprises a first substrate, a microcavity defining layer and a heating electrode wherein the microcavity defining layer is located on the first substrate, and defines a plurality of microreaction chambers; the heating electrode is located on the first substrate, is closer to the first substrate compared with the microcavity defining layer, and is configured to heat the plurality of microreaction chambers; and orthographic projections of the plurality of microreaction chambers on the first substrate are located in an orthographic projection of the heating electrode on the first substrate. The detection chip can realize a temperature cycle without performing a driving operation on liquid drops and without needing an external heating device, so that the level of integration is high, the operation is simple, and the production cost is low.

### SUMMARY

According to the present invention, which is defined in claim 1, they are provided an array substrate, a microfluidic device, microfluidic system, and a fluorescence detection method, as defined in the annexed claims.. The array substrate comprises a plurality of recesses arranged in an array. The array substrate is in a plane, and a ratio of an area of an orthographic projection of the plurality of recesses on the plane to an area of an orthographic projection of the array substrate on the plane is between 0.05 and 0.60. The array substrate further comprises a first substrate, a defining layer on the first substrate and defining the plurality of recesses, a shielding layer defining a plurality of openings, an orthographic projection of each of the plurality of openings on the first substrate at least partially overlapping an orthographic projection of a respective one of the plurality of recesses on the first substrate, and an orthographic projection of the shielding layer on the first substrate at least partially overlapping an orthographic projection of the defining layer on the first substrate; a hydrophilic layer; and a first hydrophobic layer. The hydrophilic layer covers a sidewall and a bottom of each of the plurality of recesses as well as a surface of the defining layer away from the first substrate, the first hydrophobic layer is on a side of the defining layer away from the first substrate and farther away from the first substrate than the hydrophilic layer. The first hydrophobic layer defines a plurality of holes, the plurality of holes, the plurality of recesses, and the plurality of openings correspond one by one with each other. Both a first orthographic projection of each of the plurality of holes on the first substrate and a third orthographic projection of an opening corresponding to the hole on the first substrate are within a second orthographic projection of a recess corresponding to the hole on the first substrate, and the first orthographic projection, the second orthographic projection, and the third orthographic projection form concentric rings; and the first orthographic projection is between the second orthographic projection and the third orthographic projection, and the third orthographic projection is within the first orthographic projection.

In some embodiments, the plurality of recesses penetrate the defining layer.

In some embodiments, the shielding layer is between the first substrate and the defining layer.

In some embodiments, the shielding layer is on a side of the first substrate away from the defining layer.

In some embodiments, the shielding layer comprises a first portion, the first portion is on a side of the defining layer away from the first substrate, and the first portion is attached to side surfaces of the defining layer and a surface of the defining layer away from the first substrate. The first portion defines the plurality of openings.

In some embodiments, the shielding layer further comprises a second portion, the second portion is attached to a surface of the defining layer close to the first substrate to surround the defining layer together with the first portion. The second portion defines the plurality of openings. An orthographic projection of the plurality of openings defined by the first portion of the shielding layer on the first substrate and an orthographic projection of the plurality of openings defined by the second portion of the shielding layer on the first substrate completely overlap.

In some embodiments, a surface of the defining layer close to the first substrate and/or a surface of the defining layer away from the first substrate constitute the shielding layer.

In some embodiments, the plurality of recesses correspond to the plurality of openings one by one, and an orthographic projection of each of the plurality of openings on the first substrate is within an orthographic projection of a recess corresponding to the opening on the first substrate.

In some embodiments, both a shape of an orthographic projection of each of the plurality of recesses and a shape of an orthographic projection of each of the plurality of openings on the first substrate comprise a circle or a regular polygon. The plurality of recesses correspond to the plurality of openings one by one, and an orthographic projection of each of the plurality of openings on the first substrate is within an orthographic projection of a recess corresponding to the opening on the first substrate. Both a shape of an orthographic projection of each opening and a shape of an orthographic projection of a recess corresponding to the opening on the first substrate are circular, a diameter of each opening is in a range of 20-80 µm, and a diameter of the recess corresponding to the opening is in a range of 25-90 µm; or a shape of an orthographic projection of each opening on the first substrate is a first regular polygon, and a shape of an orthographic projection of the recess corresponding to the opening on the first substrate is a second regular polygon, a diameter of an inscribed circle of the first regular polygon is in a range of 20-80 µm, and a diameter of an inscribed circle of the second regular polygon is in a range of 25-90 µm.

In some embodiments, the array substrate further comprises a heating electrode between the first substrate and the defining layer, the heating electrode is configured to heat the plurality of recesses and comprises a plurality of sub-parts separated from each other; a conductive layer between the first substrate and the heating electrode and electrically connected to the heating electrode. An orthographic projection of at least a portion of the conductive layer on the first substrate falls on a periphery of an orthographic projection of the heating electrode on the first substrate, and the conductive layer at least partially surrounds the heating electrode.

In some embodiments, a ratio of an area of the first hydrophobic layer to an area of the hydrophilic layer is between 0.01 and 2.00.

According to another aspect of the present disclosure, a microfluidic device is provided. The microfluidic device comprises the array substrate described in any of the previous embodiments, a counter substrate for assembling with the array substrate, and a space between the array substrate and the counter substrate. The counter substrate comprises a second substrate and a second hydrophobic layer on a side of the second substrate close to the first substrate. The counter substrate comprises at least one through hole penetrating the second substrate and the second hydrophobic layer. The second hydrophobic layer comprises a light-absorbing material, and the light-absorbing material comprises at least one of TiO₂ and TiON.

According to still another aspect of the present disclosure, a microfluidic system is provided. The microfluidic system comprises a control device and the microfluidic device described in any of the previous embodiments. The control device is electrically connected to the microfluidic device, and is configured to control the temperature of the microfluidic device.

According to yet another aspect of the present disclosure, a fluorescence detection method is provided. The fluorescence detection method comprises: containing a reagent to be tested in the plurality of recesses of the microfluidic device described in any of the previous embodiments; making a light of a first wavelength emitted by a light source irradiate the plurality of recesses through the plurality of openings defined by the shielding layer; and detecting a light of a second wavelength emitted by the reagent to be tested.

### BRIEF DESCRIPTION OF THE DRAWINGS

In order to more clearly describe the technical solutions in the embodiments of the present disclosure, the following will briefly introduce the drawings that need to be used in the embodiments. Obviously, the rawings in the following description are only some embodiments of the present disclosure. For those of ordinary skill in the art, other drawings can be obtained based on these drawings without creative work.
FIG. 1 illustrates a partial cross-sectional view of an array substrate according to an embodiment of the present disclosure;
FIG. 2 illustrates a partial cross-sectional view of an array substrate according to an embodiment of the present disclosure;
FIG. 3 illustrates a partial top plan view of a shielding layer and a defining layer defining recesses in an array substrate according to an embodiment of the present disclosure;
FIG. 4 illustrates a top plan view of a defining layer defining recesses in an array substrate according to an embodiment of the present disclosure;
FIG. 5 illustrates a top plan view of an array substrate according to an embodiment of the present disclosure;
FIG. 6 illustrates a top plan view of a portion of the structure in an array substrate according to an embodiment of the present disclosure;
FIG. 7 illustrates a partial cross-sectional view of an array substrate according to another embodiment of the present disclosure;
FIG. 8 illustrates a partial cross-sectional view of an array substrate according to still another embodiment of the present disclosure;
FIG. 9 illustrates a partial cross-sectional view of an array substrate according to yet another embodiment of the present disclosure;
Figure 10 illustrates a partial cross-sectional view of a microfluidic device according to an embodiment of the present disclosure;
Figure 11 illustrates a block diagram of a microfluidic system according to an embodiment of the present disclosure;
FIG. 12 illustrates a flowchart of a fluorescence detection method according to an embodiment of the present disclosure;
FIG. 13 illustrates a schematic diagram of a fluorescence detection process of a microfluidic device according to an embodiment of the present disclosure;
Fig. 14A illustrates a fluorescence picture of a microfluidic device in the related art after being irradiated by a light source; and
Fig. 14B illustrates a fluorescence picture of a microfluidic device according to an embodiment of the present disclosure after being irradiated by a light source.

### DETAILED DESCRIPTION OF THE DISCLOSURE

The technical solutions in the embodiments of the present disclosure will be clearly and completely described below in conjunction with the drawings in the embodiments of the present disclosure. Obviously, the described embodiments are only a portion of the embodiments of the present disclosure, rather than all the embodiments. Based on the embodiments in the present disclosure, all other embodiments obtained by those of ordinary skill in the art without creative work shall fall within the protection scope defined by the appended claims.

Because the dPCR technology has the advantages of high sensitivity, strong specificity, high detection throughput, accurate quantification, etc., it is widely used in various fields such as clinical diagnosis, gene instability analysis, single-cell gene expression, environmental microbial detection and prenatal diagnosis. In the application of dPCR, after the PCR amplification of the reaction system solution in each reaction chamber in the microfluidic device is completed, it is usually necessary to use an excitation light of a certain wavelength to perform fluorescence detection on the reagent to be tested in each reaction chamber. However, the inventor(s) found that in a conventional microfluidic device, since the area occupied by all reaction chambers is much smaller than the area of the microfluidic device, that is, each reaction chamber has a very small volume, the amplification reaction cannot be fully performed on the reaction system solution in each reaction chamber and a small dose of the reagent to be tested is therefore obtained. Such a dose of the reagent to be tested, which is much lower than the required dose, cannot radiate the desired fluorescence intensity under the irradiation of the excitation light, which seriously affects the fluorescence detection accuracy of the reagent to be tested in the reaction chamber. As a result, the fluorescence detection results obtained cannot meet the diagnostic requirements of the biomedical field (such as single-cell analysis, early cancer diagnosis, and prenatal diagnosis).

It should be noted that in this specification, the "reagent to be tested" refers to the reagent after the polymerase chain reaction of the reaction system solution in the microfluidic device, that is, the reaction system reagent after the amplification reaction is completed.

Based on this, the embodiments of the present disclosure provide an array substrate, a microfluidic device, a microfluidic system, and a fluorescence detection method. The array substrate can improve the fluorescence detection accuracy of the reagent to be tested in the microfluidic device comprising the array substrate.

Hereinafter, various embodiments of the present disclosure will be described in detail with reference to the accompanying drawings. It should be noted that the same reference numerals in different drawings will be used to refer to the same elements.

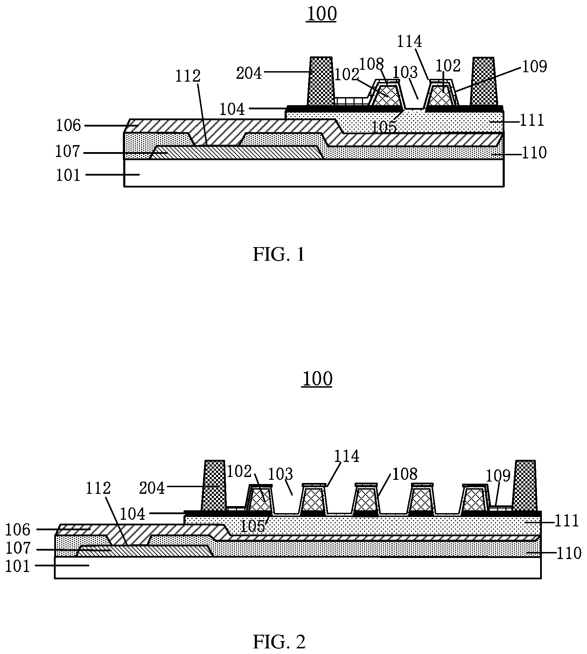

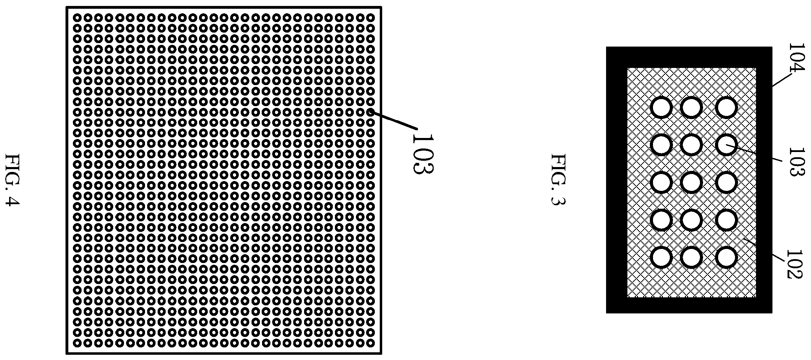

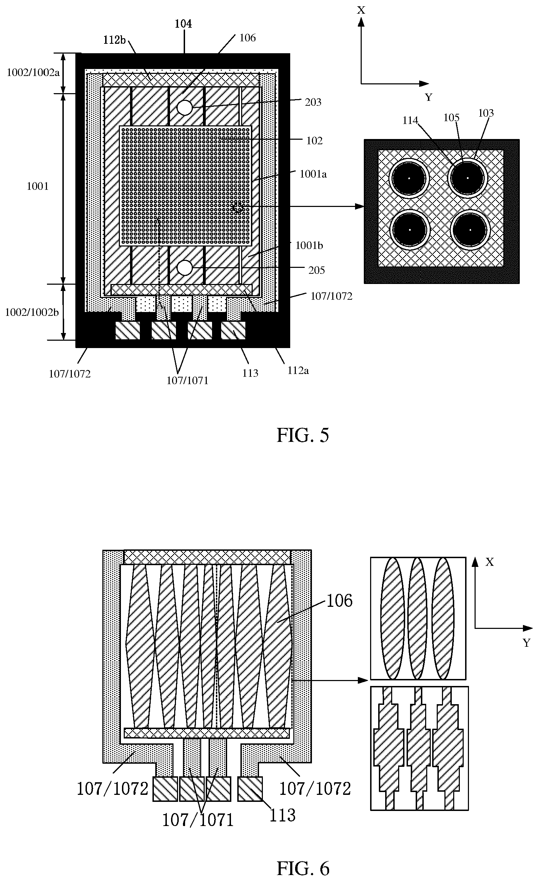

According to an aspect of the present disclosure, an array substrate 100 is provided. FIG. 1 illustrates a partial cross-sectional view of the array substrate 100, FIG. 2 illustrates another cross-sectional view of the array substrate 100 (FIG. 2 is a cross-sectional view taken along the line A-A' of FIG. 5), and FIG. 3 illustrates a top plan view of a portion of the structure in the array substrate 100. Referring to FIGS. 1 to 3, the array substrate 100 comprises a plurality of recesses 103, the array substrate 100 is located in a plane. A ratio of an area of an orthographic projection of the at least one recess 103 on the plane to an area of an orthographic projection of the array substrate 100 on the plane is between 0.05 and 0.60. For example, the ratio of the area of the orthographic projection of the at least one recess 103 on the plane to the area of the orthographic projection of the array substrate 100 on the plane may be 0.05, 0.10, 0.15, 0.20, 0.25, 0.30, 0.35, 0.40, 0.45, 0.50, 0.55, 0.60. It should be noted that, in this context, the "plane where the array substrate 100 is located" refers to the plane where the array substrate 100 as a whole is located, and the array substrate 100 extends in this plane. In some examples, the number of the at least one recess 103 may be 2,000 to 1,000,000. In some examples, the number of the recesses 103 may be 40,000 to 100,000. In one example, the number of the at least one recess 103 is 22,500. The shape of the orthographic projection of each recess 103 on the plane where the array substrate 100 is located can be any suitable shape such as a circle, an ellipse, a rectangle, a square, a triangle, a regular hexagon, a polygon (for example, a regular polygon), an irregular shape, etc.

The recesses 103 are used to contain the reaction system solution (for example, DNA molecules), and the reagent to be tested is obtained after the quantity of the reaction system solution is amplified by the dPCR technology. The inventor(s) of the present application design(s) the area ratio of the plurality of recesses 103 to the array substrate 100 so that the two have an appropriate area ratio relationship. That is, the ratio of the area of the orthographic projection of the plurality of recesses 103 on the plane where the array substrate 100 is located to the area of the orthographic projection of the array substrate 100 on the plane is between 0.05 and 0.60, so that it is possible to make the array substrate 100 have an appropriate number of recesses 103 each with an appropriate chamber volume. In this way, the dPCR reaction can be fully performed on the reaction system solution contained in each recess 103 so as to improve the utilization ratio of the reaction system solution, so that more doses of the reagent to be tested can be obtained after amplification. Such dose of the reagent to be tested can radiate a desired fluorescence intensity under the irradiation of the excitation light, which can improve the fluorescence detection accuracy of the reagent to be tested, so that the obtained fluorescence detection result can meet the diagnostic requirements of the biomedical field (for example, single cell analysis, early cancer diagnosis and prenatal diagnosis).

Continuing to refer to FIGS. 1 to 3, the array substrate 100 further comprises: a first substrate 101; a defining layer 102 on the first substrate 101 and defining the aforementioned recesses 103; and a shielding layer 104 defining a plurality of openings 105. An orthographic projection of each of the plurality of openings 105 on the first substrate 101 at least partially overlaps an orthographic projection of a respective one of the plurality of recesses 103 on the first substrate 101, and an orthographic projection of the shielding layer 104 on the first substrate 101 at least partially overlaps an orthographic projections of the defining layer 102 on the first substrate 101.

The recesses 103 are formed by patterning the defining layer 102, for example, a plurality of recesses 103 are obtained by digging the defining layer 102. In addition, it should be noted that although the defining layer 102 and the shielding layer 104 are described in this specification, the defining layer 102 and the shielding layer 104 may be two independent film structures or the same film structure. In some embodiments, the defining layer 102 and the shielding layer 104 are two independent film structures, the defining layer 102 is formed of a film layer with a suitable material, and the shielding layer 104 is formed of another film layer with light-shielding property. In an alternative embodiment, the defining layer 102 and the shielding layer 104 are the same film structure, for example, a portion of the defining layer 102 (for example, a surface of the defining layer 102 close to the first substrate 101 and/or a surface of the defining layer 102 away from the first substrate 101) is physically or chemically treated to make it opaque, so that the portion of the defining layer 102 serves as the shielding layer 104; and the remaining untreated portion of the defining layer 102 continues to play the role of the defining layer 102.

The excitation light of a certain wavelength is irradiated to the recess 103 through the opening 105 defined by the shielding layer 104 in the direction from the bottom to the top (that is, the direction from the first substrate 101 to the defining layer 102 in FIG. 1), so that the reagent with fluorescent property in the recess 103 is excited and emits a fluorescent spectrum. Due to its inherent material property, the defining layer 102 in the array substrate 100 usually emits undesirable fluorescence after being irradiated by the excitation light. However, in the embodiment of the present disclosure, by providing the shielding layer 104 and making the orthographic projection of the shielding layer 104 on the first substrate 101 at least partially overlap the orthographic projection of the defining layer 102 on the first substrate 101, when excitation light is irradiated to the recess 103 through the opening 105, the shielding layer 104 can at least partially shield the defining layer 102 from being irradiated by the excitation light, thereby preventing the defining layer 102 from being irradiated by the excitation light and causing interference fluorescence. In this way, the excitation light can only excite the reagent to be tested in the recess 103 through the opening 105. Therefore, through this arrangement, the fluorescence interference caused by the defining layer 102 can be reduced or even avoided, so that the fluorescent signal emitted by the reagent to be tested in the recess 103 can be accurately identified by the detector and the reaction signal can be read more sensitively and accurately, the fluorescence detection accuracy of the reagent to be tested can be improved, and image data support for subsequent nucleic acid amplification reaction data analysis is provided. In addition, through such an arrangement, clearer micro-hole array imaging can be achieved, detection errors caused by false positives can be reduced, and interference between different channels in the process of multi-channel fluorescence signal detection can be well avoided.

It should be noted that "a reagent with fluorescent property" means that when the reagent is irradiated by excitation light of a specific wavelength, it will emit fluorescence with a longer wavelength than the excitation light in a short period of time. Under the irradiation of the excitation light of the specific wavelength, the reagent is easier to emit fluorescence than other films in the array substrate 100.

The first substrate 101 protects and supports the array substrate 100. The first substrate 101 may be made of any suitable material, for example, a rigid material or a flexible material. The rigid or flexible material comprises but is not limited to, glass, ceramic, silicon, polyimide, and other materials. In an example, the first substrate 101 is made of glass, and the glass material can reduce the surface roughness of the first substrate 101 and facilitate the movement of the reaction solution (for example, liquid droplets) on the surface of the corresponding film layer.

The defining layer 102 can be made of any suitable material. In some embodiments, the material of the defining layer 102 is photoresist. The photoresist may be formed on the first substrate 101 by any suitable method (for example, spin coating), and be patterned to form the defining layer 102. The defining layer 102 generally has a relatively thick thickness. In an example, the thickness of the defining layer 102 may range from 5 microns to 100 microns, for example, 9.8 microns. The defining layer 102 defines a plurality of recesses 103, and the plurality of recesses 103 are spaced apart from each other. Each recess 103 may penetrate the defining layer 102 or not completely penetrate the defining layer 102. In the latter case, a portion of the defining layer 102 is formed at the bottom of the recess 103. In the embodiment of the present disclosure, the recess 103 may penetrate the defining layer 102. In this way, when the excitation light is irradiated to the recess 103 through the opening 105, it can be directly irradiated to the reagent in the recess 103, so that the fluorescence detection accuracy of the reagent can be further improved. The recess 103 provides a containing space for the reaction system solution, and the reaction system solution that moves into the recess 103 stays in the recess 103 in a relatively stable manner. For example, the recess 103 may be a groove, a notch, a micro-hole, etc., as long as it has a space capable of accommodating the reaction system solution, which is not limited in the embodiment of the present disclosure.

The shapes of the plurality of recesses 103 may be completely the same, or may be partially different. In some embodiments, the shape of the orthographic projection of each recess 103 on the first substrate 101 is a circle. The three-dimensional shape of each recess 103 is, for example, an approximate cylinder, that is, the cross section in the direction perpendicular to the first substrate 101 is an approximate rectangle and the cross section on a plane parallel to the first substrate 101 is an approximate circle. In some embodiments, the diameter of the bottom surface of the cylinder ranges from 1 micron to 100 microns, for example, from 20 microns to 50 microns, or from 50 microns to 90 microns. The height of the cylinder ranges from 5 microns to 100 microns, for example, from 30 microns to 50 microns. For example, in some examples, the diameter of the bottom surface of the cylinder is about 50 microns, the height of the cylinder is in the range of 40 to 50 microns, and the distance between the centers of two adjacent recesses 103 is about 100 microns.

The shape of the recesses 103 can be designed according to actual requirements. For example, the shape of each recess 103 can also be a truncated cone, a rectangular parallelepiped, a polygonal prism, a sphere, an ellipsoid, etc., which are not limited in the embodiments of the present disclosure. For example, the cross-sectional shape of the recess 103 on a plane parallel to the first substrate 101 may be an ellipse, a triangle, a polygon, an irregular shape, etc., and the cross-section in a direction perpendicular to the first substrate 101 may be a square, circle, parallelogram, trapezoid and other polygons.

In some embodiments, a tangent to any point on the sidewall of each recess 103 is at a certain angle to the plane where the array substrate 100 is located, and the angle is not equal to 90°. In other words, the sidewall of each recess 103 is not perpendicular to the plane where the array substrate 100 is located, but has a certain inclination. The inclination angle may be, for example, an acute angle (greater than 0° and less than 90°) or an obtuse angle (greater than 90° and less than 180°). In an example, as illustrated in FIG. 1, the sidewall of the recess 103 is at an obtuse angle to the plane where the array substrate 100 is located, and the inclination angle of the sidewall makes the area of the bottom of the recess 103 smaller than the area of the upper opening corresponding to the bottom. Of course, the shape of the recess 103 is not limited to this. In another example, the sidewall of the recess 103 is at an acute angle to the plane where the array substrate 100 is located, and the inclination angle of the sidewall makes the area of the bottom of the recess 103 larger than the area of the upper opening corresponding to the bottom. By designing the sidewall of the recesses 103 in this way, each recess 103 can obtain a larger volume in a limited space, so that each recess 103 can contain more reaction system solution. More doses of the reagent to be tested are obtained after the amplification reaction, the fluorescence emission intensity of the reagent to be tested is increased, and the fluorescence detection accuracy of the reagent to be tested can be improved.

It should be noted that all the inner walls surrounding the recess 103 can be referred to as the sidewall of the recess 103. As illustrated in FIGS. 2 and 3, the sidewall of the recess 103 is adjacent to the defining layer 102, and the height of the sidewall of the recess 103 in the direction perpendicular to the first substrate 101 is substantially the same as the height of the defining layer 102 in the direction perpendicular to the first substrate 101. The sidewall of the recess 103 and the bottom of the recess 103 constitute a reaction chamber of the recess 103 to contain the reagent to be tested. In addition, it should be noted that the sidewall of the recess 103 may be an inclined surface, an arc-shaped surface, or a curved surface with any curvature (for example, a varying curvature). The embodiment of the present disclosure does not specifically limit the shape of the sidewall of the recess 103.

FIG. 3 illustrates a top plan view of the shielding layer 104 and the defining layer 102 defining the recess 103 in FIG. 2, and FIG. 4 illustrates a top plan view of the defining layer 102 defining the recess 103 in the array substrate 100. Each small circle in FIG. 4 represents a recess 103. As illustrated in FIG. 3 and FIG. 4, a plurality of recesses 103 are uniformly distributed on the first substrate 101. For example, on the first substrate 101, a plurality of recesses 103 are arranged in an array along the horizontal direction and the vertical direction. In this way, the fluorescence images obtained during the optical detection of the microfluidic device containing the array substrate 100 in the subsequent stage can be more regular and tidy, so that the detection result can be obtained quickly and accurately. Of course, the embodiment of the present disclosure is not limited to this, and the plurality of recesses 103 may also be unevenly distributed on the first substrate 101, or arranged in other manners, which is not limited in the embodiment of the present disclosure. The number of the recesses 103 may be 2,000 to 1,000,000. In some examples, the number of the recesses 103 is 40,000 to 100,000. In an example, the number of the recesses 103 is 22,500. Therefore, the array substrate 100 has a high detection throughput.

It should be noted that in the embodiments of the present disclosure, the size and number of the recesses 103 may be determined according to actual requirements, and the size and number of the recesses 103 are related to the sizes of the first substrate 101 and the array substrate 100. When the size of the recess 103 is unchanged, the larger the number of the recesses 103, the larger the sizes of the first substrate 101 and the array substrate 100.

Since the target molecule (i.e., DNA molecule) in the reaction system solution is fully diluted, when the reaction system solution enters each recess 103, the number of the target molecule in each recess 103 is less than or equal to 1, that is, each recess 103 comprises only one target molecule or does not comprise the target molecule, so that accurate detection results can be obtained in the subsequent stage.

As illustrated in FIGS. 1 and 2, the shielding layer 104 is located between the first substrate 101 and the defining layer 102. In an example, the shielding layer 104 is attached to the bottom surface of the defining layer 102 facing the first substrate 101. In an example, the orthographic projection of the defining layer 102 on the first substrate 101 completely falls within the orthographic projection of the shielding layer 104 on the first substrate 101, as illustrated in FIG. 3. With this arrangement, the excitation light incident through the opening 105 will not irradiate the defining layer 102. The shielding layer 104 may be made of any suitable material as long as the material can shield or absorb light. The embodiment of the present disclosure does not specifically limit the material of the shielding layer 104. In some embodiments, the material of the shielding layer 104 is an opaque material, and the opaque material may be, for example, an opaque metal. In some examples, the material of the shielding layer 104 is a black matrix (BM) commonly used in the display field, and the material of the black matrix comprises one or more of chromium, chromium oxide, and black resin. In some examples, the thickness of the shielding layer 104 in a direction perpendicular to the first substrate 101 is in the range of 0.6 microns to 2.4 microns, for example, 2 microns.

The shielding layer 104 defines a plurality of openings 105, and the plurality of openings 105 correspond to the plurality of recesses 103 one by one, and the orthographic projection of each opening 105 on the first substrate 101 is within the orthographic projection of a recess 103 corresponding to the opening 105 on the first substrate 101. That is, the area of the orthographic projection of each opening 105 on the first substrate 101 is smaller than the area of the orthographic projection of a recess 103 corresponding to the opening 105 on the first substrate 101. Through this arrangement, in the subsequent fluorescence detection, the excitation light can only be irradiated to the recess 103 through the opening 105, but not to the area around the recess 103, so that the interference of the film(s) in the surrounding area near the recess 103 (for example, a portion of the defining layer 102) on the fluorescence detection can be avoided. In actual product design, the size of the opening 105 of the shielding layer 104 can be correspondingly designed according to the size of the irradiation spot of the incident excitation light, the thickness of the defining layer 102, and the like. It should be noted that terms such as "a plurality of A correspond to a plurality of B one by one" mean that the number of A is equal to the number of B, and each A corresponds to one B, and the orthographic projection of each A on the first substrate 101 at least partially overlaps the orthographic projection of a B corresponding to the A on the first substrate 101. In some embodiments, the shape of each opening 105 and the shape of the recess 103 corresponding to the opening 105 may be the same or different. The shape of the opening 105 may be a cylindrical, a truncated cone, a rectangular parallelepiped, a polygonal prism, a sphere, an ellipsoid, etc., which is not limited in the embodiments of the present disclosure. For example, the cross-sectional shape of the opening 105 on a plane parallel to the first substrate 101 may be an ellipse, a triangle, a polygon, an irregular shape, etc., and the cross-section in a direction perpendicular to the first substrate 101 may be a square, a circle, a parallelogram, a trapezoid, or other polygons. In an example, both the shape of the orthographic projection of each opening 105 and the shape of the orthographic projection of a corresponding recess 103 on the first substrate 101 are circular, and the diameter of each opening 105 is in the range of 20 to 80 microns, and the diameter of a recess 103 corresponding to the opening 105 is in the range of 25 to 90 microns. It should be noted that the diameter of the opening 105 is smaller than the diameter of the recess 103 corresponding to the opening 105. For example, if the diameter of the opening 105 is 20 microns, the diameter of the corresponding recess 103 may be 25 microns; if the diameter of the opening 105 is 60 microns, the diameter of the corresponding recess 103 may be 70 microns; if the diameter of the opening 105 is 80 microns, the diameter of the corresponding recess 103 may be 90 microns, etc. In another example, the shape of the orthographic projection of each opening 105 on the first substrate 101 is a first regular polygon, and the shape of the orthographic projection of a recess 103 corresponding to the opening 105 on the first substrate 101 is a second regular polygon, the diameter of the inscribed circle of the first regular polygon is in the range of 20 to 80 µm, and the diameter of the inscribed circle of the second regular polygon is in the range of 25 to 90 µm. It should be noted that the diameter of the inscribed circle of the first regular polygon is smaller than the diameter of the inscribed circle of the second regular polygon. For example, if the diameter of the inscribed circle of the first regular polygon is 20 microns, the diameter of the inscribed circle of the second regular polygon may be 25 microns; if the diameter of the inscribed circle of the first regular polygon is 60 microns, the diameter of the inscribed circle of the second regular polygon may be 70 microns; if the diameter of the inscribed circle of the first regular polygon is 80 microns, the diameter of the inscribed circle of the second regular polygon may be 90 microns, etc. By making the shape of the opening 105 and the recess 103 substantially the same and making the diameter of the opening 105 smaller than the diameter of the recess 103, the excitation light can be irradiated to the recess 103 at a higher utilization ratio, and as described above, only the recess 103 can be irradiated, and the surrounding area of the recess 103 will not be irradiated.

Since the shielding layer 104 is located between the first substrate 101 and the defining layer 102, the orthographic projection of the shielding layer 104 on the first substrate 101 necessarily at least partially overlaps the first substrate 101 per se, that is, the shielding layer 104 necessarily shields at least a portion of the first substrate 101. Therefore, when the excitation light is irradiated to the recess 103 through the opening 105 in the direction from bottom to top, the shielding layer 104 can not only block the excitation light from irradiating the defining layer 102, but also block the excitation light irradiated on the first substrate 101 from being transmitted through the shielding layer 104, so as not only to prevent the defining layer 102 from emitting undesired fluorescence, but also to at least partially reduce the slight fluorescence interference (possibly) generated by the first substrate 101 when it is irradiated by the excitation light. This further improves the fluorescence detection accuracy of the reagent in the recess 103.

Although the foregoing embodiment is described with the defining layer 102 and the shielding layer 104 as two separate film structures, as mentioned above, the defining layer 102 and the shielding layer 104 may also be the same film structure. In the embodiment where the defining layer 102 and the shielding layer 104 have the same film structure, the surface of the defining layer 102 close to the first substrate 101 (that is, the lower surface of the defining layer 102) is physically or chemically treated to make it opaque, so that the lower surface of the defining layer 102 serves as the shielding layer 104. The remaining untreated portion of the defining layer 102 continues to function as the defining layer 102. When the recess 103 penetrates the defining layer 102 (that is, the recess 103 is a structure similar to a through hole), the opening at the bottom of the recess 103 is equivalent to the opening 105 of the shielding layer 104; when the recess 103 does not completely penetrate the defining layer 102 (that is, the recess 103 is a structure similar to a blind hole), by processing the lower surface of the defining layer 102, an opening is formed at a position corresponding to the recess 103, which is equivalent to the opening 105 of the shielding layer 104.

During the dPCR reaction, the double-stranded structure of the DNA fragment is denatured to form a single-stranded structure at a high temperature (for example, 90°C). At a low temperature (for example, 65°C), the primer and the single strand are combined according to the principle of base complementary pairing and achieve base-binding extension at the most suitable temperature (for example, 72°C) for the DNA polymerase, the above process is the temperature cycle of denaturation-annealing-extension. Through multiple temperature cycles of denaturation-annealing-extension, DNA fragments can be replicated in large numbers.

In order to realize the above-mentioned temperature cycling process, a series of external devices are usually used to heat and cool the microfluidic device comprising the array substrate 100, which makes the device bulky, complicated to operate, and costly. Moreover, in the process of heating and cooling the microfluidic device, the overall temperature of the microfluidic device changes accordingly, so that in addition to the recess 103 which contains the DNA fragments, the temperature of other structures and components in the microfluidic device also changes. This change increases the risk of damage to components such as circuits. In addition, since tens of thousands to hundreds of thousands of recesses 103 are usually distributed in the microfluidic device, the phenomenon of uneven temperature at each recess 103 may occur during heating the microfluidic device, for example, the temperature in the middle area of the reaction area ( which is composed of the plurality of recesses 103) is high while the temperature in the edge area of the reaction area is low, which will affect the entire dPCR process and make the final detection result of the reagent inaccurate.

In order to solve the above-mentioned problems, as illustrated in FIGS. 1 and 2, the array substrate 100 further comprises a heating electrode 106 located between the first substrate 101 and the defining layer 102, and the heating electrode 106 is configured to heat the plurality of recesses 103.

The heating electrode 106 is located on the first substrate 101, and the heating electrode 106 can receive an electrical signal (such as a voltage signal), so that when a current flows through the heating electrode 106, heat is generated, and the heat is conducted to the recess 103 for polymerase chain reaction. For example, the heating electrode 106 can be made of a conductive material with a large resistivity, so that the heating electrode 106 can generate a large amount of heat when provided with a small electrical signal, so as to increase the power conversion efficiency. The heating electrode 106 may be made of, for example, a transparent conductive material, such as indium tin oxide (ITO), tin oxide, etc., or may be made of other suitable materials, such as metal, which is not limited in the embodiments of the present disclosure.

As illustrated in the figure, the orthographic projection of the plurality of recesses 103 on the first substrate 101 is located within the orthographic projection of the heating electrode 106 on the first substrate 101. Here, the orthographic projection refers to the projection on the first substrate 101 in a direction perpendicular to the first substrate 101. For example, as illustrated in FIG. 2, in the direction perpendicular to the first substrate 101, the orthographic projection of the plurality of recesses 103 on the first substrate 101 is located within the orthographic projection of the heating electrode 106 on the first substrate 101, and the orthographic projection of the heating electrode 106 is larger than the orthographic projection of the plurality of recesses 103. In this way, the heating electrode 106 can heat each recess 103. Due to the edge heat dissipation effect of the heating electrode 106, the operating temperature at the edge of the heating electrode 106 is generally lower than the operating temperature at the center area of the heating electrode 106. By making the above-mentioned projection of the heating electrode 106 larger than the above-mentioned projection of the plurality of recesses 103, the central area of the heating electrode 106 having a uniform temperature corresponds to the plurality of recesses 103 and heats the plurality of recesses 103, so as to prevent the plurality of recesses 103 from being heated by the edge of the heating electrode 106 (for example, an area 5 mm, 8 mm or other suitable size from the edge), so that the heating of the plurality of recesses 103 is more uniform and the temperature uniformity is better, which in turn facilitates the effective amplification reaction of the reaction system solution in the recesses 103.

FIG. 5 illustrates a top plan view of the array substrate 100. As illustrated in the figure, the heating electrode 106 comprises a plurality of sub-parts which are separated from each other and extending in a first direction X (i.e., the vertical direction in the figure). The figure illustrates five sub-parts which are separated and insulated from each other, but the embodiments of the present disclosure are not limited to this, the heating electrode 106 may comprise more or less separated sub-parts, such as two separated sub-parts, ten separated sub-parts, one hundred separated sub-parts, etc. The embodiments of the present disclosure do not limit the spacing between the adjacent sub-parts, as long as the product design requirements are met. In an example, the spacing between each sub-part of the heating electrode 106 is in the range of 1 micron to 200 microns. By applying electrical signals to each sub-part of the heating electrode 106 respectively, for example, applying a high voltage to one end of each sub-part, and applying a low voltage (for example, a ground signal) to the other end of each sub-part, the separated sub-parts of the heating electrode 106 flow substantially the same amount of current, and therefore maintain substantially the same temperature, thereby further improving the temperature uniformity of the heating electrode 106, so that the heating of the plurality of recesses 103 is more uniform, and the temperature uniformity is better, which in turn facilitates the amplification reaction of the reaction system solution in the recesses 103.

In FIG. 5, each sub-part of the heating electrode 106 is illustrated as an elongated rectangle, but the embodiment of the present disclosure does not limit the shape of each sub-part of the heating electrode 106, which may be any appropriate shape. For example, FIG. 6 illustrates another possible planar shape of each sub-part of the heating electrode 106. As illustrated in the figure, the heating electrode 106 comprises a plurality of strip-like structures that extend along the first direction X and are separated from each other. The width of the middle part of each strip structure in the second direction Y is wider than the width of the two end parts of the strip structure in the second direction Y. The second direction Y is a direction perpendicular to the first direction X in a plane parallel to the first substrate 101. Of course, the plurality of strip structures in the heating electrode 106 are not limited to the shapes illustrated at the left side of FIG. 6, as long as the strip structure has a shape such that the width of the middle portion is wider than the width of the two end portions. For example, as illustrated at the right side of FIG. 6, the shape of the strip structure of the heating electrode 106 may also be an ellipse or a rectangle with an edge profile in the shape of a broken line.

In the embodiment of the present disclosure, by arranging the heating electrode 106 in the array substrate 100 (for example, integrating the heating electrode 106 on the first substrate 101), the heating of the recess 103 of the array substrate 100 can be effectively realized, thereby realizing the temperature control of the recess 103 without external heating equipment and with high integration. By arranging the heating electrode 106 as a plurality of sub-parts separated from each other, the heating electrode 106 can be maintained at substantially the same temperature everywhere, thereby further improving the temperature uniformity of the heating electrode 106 and making the heating of the plurality of recesses 103 more uniform. In addition, compared to some array substrates that need to drive liquid droplets to move through regions of multiple temperatures in order to be heated, the array substrate 100 can realize temperature cycling without driving the liquid droplets, and has simple operation and low production cost.

Returning to continue referring to FIGS. 1 and 2, the array substrate 100 further comprises a conductive layer 107 and a first insulating layer 110. The conductive layer 107 is located between the first substrate 101 and the heating electrode 106, and the first insulating layer 110 is located between the conductive layer 107 and the heating electrode 106, the conductive layer 107 is electrically connected to the heating electrode 106 through the via 112 in the first insulating layer 110. The conductive layer 107 is configured to apply an electric signal (for example, a voltage signal) to the heating electrode 106. After the heating electrode 106 receives the electrical signal, it can generate heat under the action of the electrical signal, thereby heating the recess 103. It should be noted that the first insulating layer 110 may also cover a portion of the first substrate 101 that is not overlapped by the conductive layer 107.

The via 112 exposes a portion of the conductive layer 107 so that the heating electrode 106 can be electrically connected to the conductive layer 107 through the via 112. The shape of the via 112 may be cylindrical, truncated cone, or the like. For example, the conductive layer 107 may be electrically connected to the heating electrode 106 through one or more vias 112. When the electrical connection is achieved through a plurality of vias 112, the connection resistance can be effectively reduced, and the energy loss can be reduced. When the electrical connection is achieved through one via 112, the production process can be simplified.

The number of conductive layer 107 may be one or more, which is not limited in the embodiment of the present disclosure. When a plurality of conductive layers 107 are used to apply electrical signals to the heating electrode 106, different portions of the heating electrode 106 can receive the electrical signal at the same time, so that substantially the same amount of current flows through each position of the heating electrode 106, so the heating is more uniform. For example, when there are a plurality of conductive layers 107, the first insulating layer 110 may comprise a plurality of vias 112, and each via 112 exposes a portion of the conductive layer 107, so that the heating electrode 106 is electrically connected to the plurality of conductive layers 107 respectively through the plurality of vias 112. For example, the plurality of conductive layers 107 and the plurality of vias 112 correspond one by one. For another example, the number of the plurality of vias 112 may also be greater than the number of the plurality of conductive layers 107, and each conductive layer 107 is electrically connected to the heating electrode 106 through one or more vias 112.

It should be noted that in the embodiments illustrated in FIGS. 1 and 2, the heating electrode 106 and the conductive layer 107 are located in different layers. In some other embodiments, the heating electrode 106 and the conductive layer 107 may also be located in the same layer. In this case, the first insulating layer 110 may be omitted from the array substrate 100, and the heating electrode 106 is directly electrically connected to the conductive layer 107.

The resistance value of the heating electrode 106 is greater than the resistance value of the conductive layer 107, so that under the same electrical signal, the heating electrode 106 generates more heat to heat the recess 103. The conductive layer 107 generates less heat, thereby reducing energy loss. For example, the conductive layer 107 may use a material with a small resistivity, so as to reduce the energy loss on the conductive layer 107. The conductive layer 107 may be made of a metal material. The metal material may be copper or copper alloy, aluminum or aluminum alloy, etc., and may be a single metal layer or a composite metal layer, which is not limited in the embodiments of the present disclosure.

In some embodiments of the present disclosure, the heating electrode 106 is made of indium tin oxide (ITO) or tin oxide, and the conductive layer 107 is made of a metal material. Since ITO is not easily oxidized, it can prevent the oxidation of the portion of the heating electrode 106 exposed to the air, thereby avoiding problems such as uneven heating or increased power consumption caused by the oxidation of the heating electrode 106. The conductive layer 107 is covered by the first insulating layer 110, so even if it is made of a metal material, the problem of oxidation is unlikely to occur.

Referring to FIG. 5, the vias 112 in the first insulating layer 110 comprise a first plurality of vias 112a and a second plurality of vias 112b. The first set of vias 112a and the second set of vias 112b are respectively located on opposite sides of the array substrate 100. The conductive layer 107 comprises a first set of conductive layers 1071 and a second set of conductive layers 1072. The first set of conductive layers 1071 and the first set of vias 112a are located on the same side. The first set of conductive layers 1071 are electrically connected to the heating electrode 106 through the first set of vias 112a. The orthographic projection of the second set of conductive layers 1072 on the first substrate 101 falls on the periphery of the orthographic projection of the heating electrode 106 on the first substrate 101, and the second set of conductive layers 1072 at least partially surround the heating electrode 106. The second set of conductive layers 1072 are electrically connected to the heating electrode 106 through the second set of vias 112b. Through this arrangement, the conductive layer 107 can at least partially surround the heating electrode 106, the heat loss of the heating electrode 106 can be reduced, the temperature of each recess 103 can be more uniform, and the heating efficiency of the heating electrode 106 can be improved, thereby reducing the power consumption.

In a conventional array substrate, a high voltage signal is usually applied to the heating electrode through only one conductive layer, and a low voltage signal (such as a ground voltage) is applied to the heating electrode through the other conductive layer, so as to form, for example, a current path along the first direction on the heating electrode, which causes the heating electrode to generate heat. Since the heating electrode itself has a large resistance value, a large voltage drop will be generated at the junction between the heating electrode and the conductive layer in the second direction perpendicular to the first direction, so that the heating electrode can be divided into a first electrode and a second electrode distributed in the second direction. The first electrode receives a relatively large voltage signal. The first electrode is, for example, the part of the electrode at the junction of the heating electrode and the conductive layer. The second electrode receives a relatively small voltage signal. The second electrode is, for example, the part of the electrode that is away from the above-mentioned junction in the second direction. Correspondingly, the current in the heating electrode is not uniform, the current in the first electrode is relatively large and the heat generated is relatively large, and the current in the second electrode is relatively small and the heat generated is relatively small. Therefore, when such heating electrode is used to heat the recesses in the array substrate, the recesses at different positions have different temperatures, which ultimately affects the amplification reaction of the reaction system solution in the recesses, and hence affects the accuracy of the detection effect.

In the embodiment of the present disclosure, the first plurality of conductive layers 1071 (two are illustrated in the figure) apply a first voltage signal (for example, a high voltage signal) to the heating electrode 106 through the first set of vias 112a. The second plurality of conductive layers 1072 (two are illustrated in the figure) apply a second voltage signal (for example, a ground signal) to the heating electrode 106 through the second set of vias 112b to form a current path on the heating electrode 106. By arranging such a plurality of sets of conductive layers and each set containing a plurality of conductive layers, and in combination with the heating electrode 106 which is divided into a plurality of separate sub-parts, it is possible to make one end of each sub-part of the heating electrode 106 (an end close to the first set of vias 112a) be simultaneously applied with the same first voltage signal, and the other end of each sub-part of the heating electrode 106 (an end close to the second set of vias 112b) be simultaneously applied with the same second voltage signal, thereby a uniform current is formed in the heating electrode 106 and uniform heat is generated, so that the recesses 103 at different positions reach a uniform temperature, the amplification reaction of the reaction system solution in the recesses 103 is promoted, and the accuracy of the detection effect is improved.

In order to facilitate the electrical connection of the conductive layer 107 with an external device (not illustrated) independent of the array substrate 100 to receive electrical signals (such as voltage signals), the array substrate 100 may further comprise a contact 113 (as illustrated in FIG. 5, for example, the pad area), the contact 113 is not covered by the first insulating layer 110. For example, the contact 113 has a large square shape, so that it can be easily contacted and connected with a probe or electrode in an external device, and has a large contact area, so it can stably receive electrical signals. In this way, the array substrate 100 can be plug and play, simple to operate, and convenient to use. For example, the contact 113 may be located on the same layer as the conductive layer 107 and formed by a single patterning process. When the conductive layer 107 is made of a metal material, the contact 113 can be electroplated, thermal sprayed, or vacuum-plated to form a metal protective layer on the surface of the contact 113 to prevent the contact 113 from being oxidized without affecting its electrical conductivity.

Continuing to refer to FIG. 5, the array substrate 100 comprises a reaction area 1001 and a peripheral area 1002, and the peripheral area 1002 at least partially surrounds the reaction area 1001. In some embodiments, in the first direction X, the peripheral area 1002 comprises a first sub-area 1002a and a second sub-area 1002b respectively located on both sides of the reaction area 1001. In other embodiments, the peripheral area 1002 completely surrounds the reaction area 1001, that is, the peripheral area 1002 is ring-shaped and surrounds the reaction area 1001. In this case, in the first direction X, the peripheral area 1002 comprises a first sub-area 1002a and a second sub-area 1002b respectively located on both sides of the reaction area 1001, and in the second direction Y, the peripheral area 1002 also comprises a third sub-area and a fourth sub-area respectively located on both sides of the reaction area 1001, the first sub-area 1002a is connected with the third sub-area and the fourth sub-area, and the second sub-area 1002b is also connected with the third sub-area and the fourth sub-area, so that the peripheral area 1002 surrounds the reaction area 1001.

As illustrated in FIG. 5, the vias 112 are located in the peripheral area 1002, and the heating electrode 106 is at least partially located in the reaction area 1001. In some embodiments, the reaction area 1001 further comprises a functional area 1001a, and the recesses 103 are located in the functional area 1001a. For example, the orthographic projection of the heating electrode 106 on the first substrate 101 completely covers the functional area 1001a of the reaction area 1001, that is, the functional area 1001a is located within the orthographic projection of the heating electrode 106 on the first substrate 101, thereby ensuring that the heating electrode 106 can heat each recess 103.

It should be noted that when the peripheral area 1002 further comprises the third sub-area and the fourth sub-area located on both sides of the reaction area 1001 in the second direction Y, the third sub-area and the fourth sub-area may also provide a plurality of conductive layers 107. The embodiments of the present disclosure do not impose restrictions on the number, placement positions, etc. of the conductive layers 107.

Returning to continue referring to FIGS. 1 and 2, the array substrate 100 further comprises a hydrophilic layer 108 covering the sidewall of each recess 103. The hydrophilic layer 108 not only covers the sidewall of each recess 103, but also covers the surface of the defining layer 102 away from the first substrate 101 and the bottom of each recess 103. In the embodiment where the recess 103 penetrates the defining layer 102 (that is, the recess 103 is a through-hole structure), the hydrophilic layer 108 covers the sidewall of the recess 103. In the embodiment where the recess 103 does not completely penetrate the defining layer 102 (that is, the recess 103 is a blind hole structure), the hydrophilic layer 108 covers the sidewall and bottom of the recess 103. The hydrophilic layer 108 has the characteristics of being hydrophilic and oleophobic. Since the sidewall (and bottom) of the recess 103 is provided with the hydrophilic layer 108, the hydrophilicity of the recess 103 is greatly improved, and the contact angle between the droplets in the reaction system solution and the surface of the recess 103 is relatively small. When no driving force is exerted on the reaction system solution from the outside, the reaction system solution can automatically gradually enter each recess 103 based on the capillary phenomenon, thereby realizing automatic sampling and avoiding cross-flow.

In some embodiments, the material of the hydrophilic layer 108 is silicon oxide, such as silicon dioxide (SiO₂). Of course, the embodiments of the present disclosure are not limited to this, and the hydrophilic layer 108 can also be made of other suitable inorganic or organic materials, as long as the surface of the hydrophilic layer 108 away from the defining layer 102 is hydrophilic. In some embodiments, the hydrophilic layer 108 may be directly prepared using hydrophilic materials. In other embodiments, the hydrophilic layer 108 may be made of a material that is not hydrophilic. In this case, it is necessary to perform a hydrophilic treatment on the surface of the hydrophilic layer 108 away from the defining layer 102, so that the surface of the hydrophilic layer 108 away from the defining layer 102 has hydrophilicity. If a non-hydrophilic material (such as silicon nitride, etc.) is used to prepare the hydrophilic layer 108, the non-hydrophilic material can be hydrophilized, for example, gel modification, ultraviolet radiation, plasma method and other methods may be selected to make the surface of the non-hydrophilic material have a hydrophilic group, thereby having hydrophilicity.

As illustrated in FIGS. 1 and 2, the array substrate 100 also comprises a first hydrophobic layer 109 on a side of the hydrophilic layer 108 away from the first substrate 101, and a portion of the hydrophilic layer 108 (that is, the portion of the hydrophilic layer 108 that covers the surface of the defining layer 102 away from the first substrate 101) is located between the first hydrophobic layer 109 and the defining layer 102. The first hydrophobic layer 109 covers the surface of the hydrophilic layer 108 (on the defining layer 102) away from the first substrate 101 and extends to the boundary between the surface and the sidewall of the recess 103, so that the first hydrophobic layer 109 defines a plurality of holes 114.

The plurality of recesses 103 defined by the defining layer 102, the plurality of openings 105 defined by the shielding layer 104, and the plurality of holes 114 defined by the first hydrophobic layer 109 correspond one by one with each other, and both the first orthographic projection of a hole 114 on the first substrate 101 and the third orthographic projection of an opening 105 corresponding to the hole 114 on the first substrate 101 are located within the second orthographic projection of a recess 103 corresponding to the hole 114 on the first substrate 101, and the first orthographic projection, the second orthographic projection, and the third orthographic projection form concentric rings, the first orthographic projection is located between the second orthographic projection and the third orthographic projection, and the third orthographic projection is located within the first orthographic projection, a concentric ring pattern as illustrated at the right side of Figure 5 is formed. That is, the size of the circular opening 105 defined by the shielding layer 104 is the smallest, the size of the circular recess 103 defined by the defining layer 102 is the largest, and the size of the circular hole 114 defined by the first hydrophobic layer 109 is between the two. Of course, the recess 103, the opening 105, and the hole 114 are not limited to forming concentric circular rings. They can also form, for example, concentric rectangular rings, concentric square rings, concentric elliptical rings, concentric polygonal rings, and the like.

The first hydrophobic layer 109 has hydrophobic and lipophilic characteristics, and the material of the first hydrophobic layer 109 is resin or silicon nitride, for example, it may be a commercially available epoxy resin with the model number DL-1001C. The first hydrophobic layer 109 may also be made of other suitable inorganic or organic materials, as long as the first hydrophobic layer 109 is hydrophobic. In some embodiments, the first hydrophobic layer 109 may be directly prepared using a hydrophobic material. In other embodiments, the first hydrophobic layer 109 may be made of a material that does not have hydrophobicity. In this case, hydrophobization treatment needs to be applied to the surface of the first hydrophobic layer 109, so that the surface of the first hydrophobic layer 109 is hydrophobic.

In the embodiment of the present disclosure, the hydrophilic layer 108 and the first hydrophobic layer 109 can jointly adjust the surface contact angle of the droplets of the reaction system solution, so that the microfluidic device containing the array substrate 100 can realize self-absorption liquid sampling and oil sealing. In the array substrate 100, the first hydrophobic layer 109 is provided to improve the hydrophobic performance of the outside of the recess103, and the hydrophilic layer 108 is provided to improve the hydrophilic performance of the inside of the recess103 (the sidewall (and bottom) of the recess 103), thereby facilitating the infiltration of the reaction system solution from the outside of the recess 103 to the inside of the recess 103. Therefore, under the joint action of the hydrophilic layer 108 and the first hydrophobic layer 109, the reaction system solution is more likely to enter each recess 103.

In some embodiments, the ratio of the area of the first hydrophobic layer 109 to the area of the hydrophilic layer 108 is between 0.01 and 2.00. For example, the ratio of the area of the first hydrophobic layer 109 to the area of the hydrophilic layer 108 may be 0.01, 0.05, 0.10, 0.50, 1.00, 1.20, 1.40, 1.60, 1.80, 2.00, etc. If the area of the first hydrophobic layer 109 is too large and the area of the hydrophilic layer 108 is too small, the reaction system solution injected into the microfluidic device from the outside is likely to adhere to the surface of the first hydrophobic layer 109 and is difficult to enter the recess 103. In the embodiment of the present disclosure, by making the first hydrophobic layer 109 and the hydrophilic layer 108 have a suitable area ratio, under the joint action of the hydrophilic layer 108 and the first hydrophobic layer 109, it is easier for the reaction system solution to enter each recess 103, thereby avoiding waste of the reaction system solution and improving the utilization ratio, thereby increasing the fluorescence emission intensity of reagent to be tested in each recess 103, and in turn improving the fluorescence detection accuracy of reagent to be tested.

As illustrated in FIGS. 1 and 2, the array substrate 100 may further comprise a second insulating layer 111, and the second insulating layer 111 is located between the heating electrode 106 and the shielding layer 104. The second insulating layer 111 is used to protect the heating electrode 106, provide insulation, prevent liquid from corroding the heating electrode 106, slow down the aging of the heating electrode 106, and can play a flattening effect. When the recess 103 penetrates the defining layer 102, the bottom of the recess 103 exposes a part of the surface of the second insulating layer 111, and the hydrophilic layer 108 covers the sidewall of the recess 103 and the exposed surface of the second insulating layer 111.

In some embodiments, the first insulating layer 110 and the second insulating layer 111 may be made of the same insulating material, for example, made of an inorganic insulating material or an organic insulating material. In an example, the material of the first insulating layer 110 and the second insulating layer 111 comprises silicon dioxide, silicon nitride, or the like.

It should be noted that although the figure illustrates that the shielding layer 104 is located between the defining layer 102 and the second insulating layer 111, this is only an example. As mentioned above, the shielding layer 104 may be located in any film layer between the first substrate 101 and the defining layer 102 or in other positions as described later. For example, the shielding layer 104 may be located between the first substrate 101 and the conductive layer 107, between the conductive layer 107 and the first insulating layer 110, between the first insulating layer 110 and the heating electrode 106, or between the heating electrode 106 and the second insulating layer 111, and so on.

In the embodiment of the present disclosure, by providing the shielding layer 104 with the opening 105, the shielding layer 104 at least partially shields the first substrate 101 and the defining layer 102, thereby avoiding or at least reducing the background fluorescence interference caused by the defining layer 102 and the first substrate 101 and improving the fluorescence detection accuracy of reagent to be tested in the recess 103.By arranging the heating electrode 106 with a plurality of separated sub-parts and optimizing the arrangement of the conductive layer 107, the temperature uniformity of the heating electrode 106 is improved, thereby facilitating the amplification reaction of the reaction system solution in the recess 103; by providing the hydrophilic layer 108 and the first hydrophobic layer 109, the reaction system solution can more easily enter each recess 103, thereby improving reaction efficiency and avoiding cross-flow.

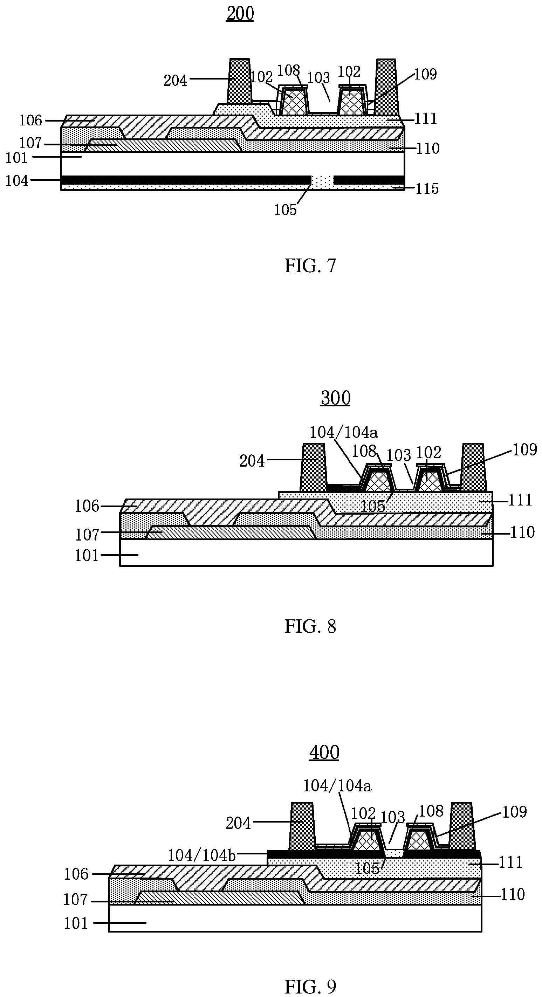

FIG. 7 illustrates a partial cross-sectional view of an array substrate 200 according to another embodiment of the present disclosure. As illustrated in the figure, except for the arrangement of the shielding layer 104 and the additional third insulating layer 115, the array substrate 200 is basically the same as the array substrate 100 illustrated in FIGS. 1 and 2. The shielding layer 104 and the third insulating layer 115 in the array substrate 200 will be described below. For other structures and corresponding technical effects, reference may be made to the array substrate 100 illustrated in FIGS. 1 and 2, which will not be repeated herein.

As illustrated in the figure, the shielding layer 104 is located on a side of the first substrate 101 away from the defining layer 102, that is, on the back side of the first substrate 101. The shielding layer 104 defines at least one opening 105, the orthographic projection of the at least one opening 105 on the first substrate 101 at least partially overlaps the orthographic projection of the at least one recess 103 on the first substrate 101, and the orthographic projection of the shielding layer 104 on the first substrate 101 at least partially overlaps the orthographic projection of the defining layer 102 on the first substrate 101. It should be noted that the figure only schematically illustrates one opening 105 defined by the shielding layer 104 and one recess 103 defined by the defining layer 102, but as described above, the shielding layer 104 actually defines a plurality of openings 105, and the defining layer 102 defines a plurality of recesses 103, and each opening 105 corresponds to each recess 103. In an example, the shape of the orthographic projection of each opening 105 on the first substrate 101 is a circle, the shape of the orthographic projection of each recess 103 on the first substrate 101 is also a circle, and the diameter of the opening 105 is smaller than the diameter of a corresponding recess 103. In the subsequent optical detection process, the excitation light may be incident into the recess 103 from the side of the first substrate 101 through the opening 105 of the shielding layer 104. The shielding layer 104 is provided on the side of the first substrate 101 away from the defining layer 102, the arrangement of the shielding layer 104 is no longer limited to other layers in the array substrate 200. The shielding layer 104 can occupy a larger area, which is beneficial for the shielding layer 104 to shield a larger part of the first substrate 101 and the defining layer 102 or to completely shield the first substrate 101 and the defining layer 102, thereby further reducing or even avoiding the background fluorescence generated by the defining layer 102 and the first substrate 101, and improving the fluorescence detection accuracy of the reagent to be tested in the recess 103.

As illustrated in the figure, the array substrate 200 further comprises a third insulating layer 115 located on a side of the shielding layer 104 away from the first substrate 101. The third insulating layer 115 covers the shielding layer 104 to protect the shielding layer 104 from the influence of external environment. The figure illustrates that the third insulating layer 115 is a continuous film layer and completely fills the opening 105 of the shielding layer 104. In another example, the third insulating layer 115 is broken at a position corresponding to the opening 105, and a film layer of the same material as the third insulating layer 115 fills the opening 105 of the shielding layer 104. In still another example, the third insulating layer 115 is also a continuous film layer but does not fill the opening 105 of the shielding layer 104. The first insulating layer 110, the second insulating layer 111, and the third insulating layer 115 may be made of the same insulating material, for example, made of an inorganic insulating material or an organic insulating material. In an example, the materials of the first insulating layer 110, the second insulating layer 111, and the third insulating layer 115 comprise silicon dioxide, silicon nitride, or the like.

FIG. 8 illustrates a partial cross-sectional view of an array substrate 300 according to another embodiment of the present disclosure. As illustrated in the figure, except for the arrangement of the shielding layer 104 and the hydrophilic layer 108, the array substrate 300 is basically the same as the array substrate 100 illustrated in FIGS. 1 and 2. The shielding layer 104 and the hydrophilic layer 108 in the array substrate 300 will be described below. For other structures and corresponding technical effects, reference may be made to the array substrate 100 illustrated in FIG. 1 and FIG. 2, which will not be repeated herein.

As illustrated in the figure, the shielding layer 104 comprises a first portion 104a, which is located on a side of the defining layer 102 away from the first substrate 101. The first portion 104a is attached to the side surfaces of the defining layer 102 and the surface of the defining layer 102 away from the first substrate 101. It should be noted that terms such as "A is attached to B" in this application refer to the direct contact between the surfaces of A and B. That is, the first portion 104a of the shielding layer 104 is in direct contact with the side surfaces of the defining layer 102 and the surface of the defining layer 102 away from the first substrate 101, and at least partially surrounds the defining layer 102. The first portion 104a of the shielding layer 104 defines a plurality of openings 105, and the orthographic projection of each opening 105 on the first substrate 101 at least partially overlaps the orthographic projection of a corresponding recess 103 on the first substrate 101. In an example, the shape of the orthographic projection of each opening 105 on the first substrate 101 is a circle, the shape of the orthographic projection of each recess 103 on the first substrate 101 is also a circle. The diameter of the opening 105 is smaller than the diameter of a corresponding recess 103. Through this arrangement, in the subsequent optical detection process, the excitation light can be directly irradiated to the reagent to be tested in the recess 103 from above the array substrate 300 through the opening 105 of the shielding layer 104 (that is, in the direction from top to bottom in the figure), which can increase the utilization ratio of the light source and increase the fluorescence emission intensity of the reagent to be tested. The side surfaces of the defining layer 102 and the surface of the defining layer 102 away from the first substrate 101 are completely shielded by the first portion 104a of the shielding layer 104, and therefore will not be irradiated by the excitation light.

Although the foregoing embodiment is described in terms of the defining layer 102 and the first portion 104a of the shielding layer 104 as two separate film structures, as mentioned above, the defining layer 102 and the first portion 104a of the shielding layer 104 may also be the same film structure. In the embodiment where the defining layer 102 and the first portion 104a of the shielding layer 104 are of the same film structure, for example, the side surfaces of the defining layer 102 and the surface of the defining layer 102 away from the first substrate 101 (i.e., the upper surface) are physically or chemically treated to make it opaque, so that the side and upper surfaces of the defining layer 102 serve as the first portion 104a of the shielding layer 104; and the remaining untreated portion of the defining layer 102 continues to function as the defining layer 102.

The hydrophilic layer 108 is disposed on the surface of the first portion 104a of the shielding layer 104 away from the first substrate 101, and covers the surface of the first portion 104a of the shielding layer 104 and at least the sidewall of each recess 103. The hydrophilic layer 108 has the characteristics of being hydrophilic and oleophobic. Since the hydrophilic layer 108 covers at least the sidewall of the recess 103, the hydrophilicity of the recess 103 can be improved. The reaction system solution can automatically and gradually enter each recess 103 based on the capillary phenomenon when no driving force is applied to the reaction system solution from the outside, thereby realizing automatic sampling and avoiding cross-flow.

FIG. 9 illustrates a partial cross-sectional view of an array substrate 400 according to yet another embodiment of the present disclosure. As illustrated in the figure, except for the arrangement of the shielding layer 104 and the hydrophilic layer 108, the array substrate 400 is basically the same as the array substrate 100 shown in FIGS. 1 and 2. The shielding layer 104 and the hydrophilic layer 108 in the array substrate 400 will be described below. For other structures and corresponding technical effects, reference may be made to the array substrate 100 illustrated in FIG. 1 and FIG. 2, which will not be repeated herein.

As illustrated in the figure, the shielding layer 104 comprises a first portion 104a and a second portion 104b. The first portion 104a is located on the side of the defining layer 102 away from the first substrate 101, and is attached to the side surfaces of the defining layer 102 and the surface of the defining layer 102 away from the first substrate 101; the second portion 104b is located between the second insulating layer 111 and the defining layer 102, and is attached to the surface of the defining layer 102 close to the first substrate 101 (i.e., the bottom surface). The first portion 104a and the second portion 104b together surround the defining layer 102. The first portion 104a of the shielding layer 104 defines a plurality of openings 105, and the orthographic projection of each opening 105 on the first substrate 101 at least partially overlaps the orthographic projection of a corresponding recess 103 on the first substrate 101. The second portion 104b of the shielding layer 104 defines a plurality of openings 105, and the orthographic projection of each opening 105 on the first substrate 101 at least partially overlaps the orthographic projection of a corresponding recess 103 on the first substrate 101. Through this arrangement, in the subsequent optical detection process, the excitation light can be either directly irradiated to the reagent to be tested in the recess 103 from above the array substrate 400 through the opening 105 of the first portion 104a of the shielding layer 104 (that is, in the direction from top to bottom in the figure), or irradiated to the recess 103 from below the array substrate 400 through the opening 105 of the second portion 104b of the shielding layer 104 (that is, in the direction from bottom to top in the figure), so that the position of the light source emitting the excitation light can be arranged flexibly according to needs. Since all surfaces (upper surface, lower surface, and each side surface) of the defining layer 102 are completely shielded by the shielding layer 104, the defining layer 102 will not be irradiated by the excitation light.

In an example, the orthographic projection of the opening 105 defined by the first portion 104a of the shielding layer 104 on the first substrate 101 completely overlaps the orthographic projection of the opening 105 defined by the second portion 104b of the shielding layer 104 on the first substrate 101. For example, the shape of the orthographic projection of the opening 105 defined by the first portion 104a of the shielding layer 104 on the first substrate 101 is circular, and the shape of the orthographic projection of the opening 105 defined by the second portion 104b of the shielding layer 104 on the first substrate 101 is also circular. The sizes of the openings respectively defined by the first portion 104a and the second portion 104b are exactly the same. In another example, the shape of the orthographic projection of the recess 103 on the first substrate 101 is circular, the shape of the orthographic projection of the opening 105 defined by the first portion 104a of the shielding layer 104 on the first substrate 101 is circular, and the shape of the orthographic projection of the opening 105 defined by the second portion 104b of the shielding layer 104 on the first substrate 101 is also circular. The openings respectively defined by the first portion 104a and the second portion 104b have the same size and are both smaller than the size of the recess 103.

Although the foregoing embodiment is described in terms of the defining layer 102 and the first portion 104a and the second portion 104b of the shielding layer 104 as separate film layer structures, as described above, the defining layer 102 and the first portion 104a and the second portion 104b of the shielding layer 104 may also be the same film structure. In the embodiment in which the defining layer 102 and the first portion 104a and the second portion 104b of the shielding layer 104 are of the same film structure, for example, the side surfaces of the defining layer 102, the surface of the defining layer 102 away from the first substrate 101 (that is, the upper surface) and the surface of the defining layer 102 close to the first substrate 101 (that is, the lower surface) are physically and chemically treated to make it opaque, thereby making the side surfaces, upper surface, and lower surface of the defining layer 102 (that is, all outer surfaces of the defining layer 102) serve as the first portion 104a and the second portion 104b of the shielding layer 104; and the remaining untreated portion of the defining layer 102 (for example, the inner portion of the defining layer 102 wrapped by the outer surface) continues to function as the defining layer 102.

The hydrophilic layer 108 is disposed on the surface of the first portion 104a of the shielding layer 104 away from the first substrate 101, and covers the surface of the first portion 104a of the shielding layer 104 and at least covers the sidewall of each recess 103. The hydrophilic layer 108 has the characteristics of being hydrophilic and oleophobic. Since the hydrophilic layer 108 covers at least the sidewall of the recess 103, the hydrophilicity of the recess 103 can be improved. The reaction system solution can automatically and gradually enter each recess 103 based on the capillary phenomenon when no driving force is applied to the reaction system solution from the outside, thereby realizing automatic sampling and avoiding cross-flow.

According to another aspect of the present disclosure, a microfluidic device is provided. The microfluidic device comprises the array substrate described in any of the previous embodiments. The following takes the microfluidic device comprising the array substrate 100 as an example to introduce. It should be noted that the array substrate provided herein can be used not only in the field of microfluidics, but also in any other appropriate fields, such as the display field, the automobile field, and the like.

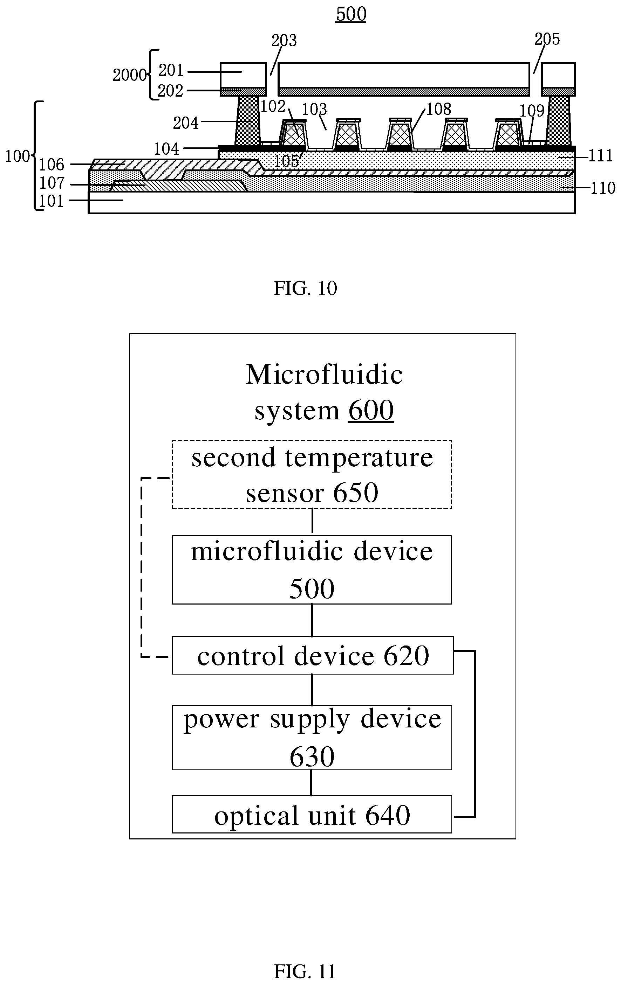

FIG. 10 illustrates a microfluidic device 500 which comprises the array substrate 100, a counter substrate 2000 for assembling with the array substrate 100, and a space between the array substrate 100 and the counter substrate 2000. The counter substrate 2000 comprises a second substrate 201 and a second hydrophobic layer 202, and the second hydrophobic layer 202 is located on a side of the second substrate 201 close to the first substrate 101. The counter substrate 2000 comprises at least one through hole penetrating the second substrate 201 and the second hydrophobic layer 202. In some embodiments, the size of the microfluidic device 500 is 1.5 cm*1.5 cm.

In an example, both the first substrate 101 and the second substrate 201 are glass substrates. The second substrate 201 is disposed opposite to the first substrate 101, and plays a role of protection, support, isolation, and the like. The microfluidic device 500 is prepared by a glass-based microfabrication method combined with a semiconductor process, so that mass production can be realized, and the corresponding production cost can be greatly reduced. It should be noted that in multiple embodiments of the present disclosure, the first substrate 101 and the second substrate 201 may also use other suitable materials, which are not limited in the embodiments of the present disclosure.

In some embodiments, the shape of the first substrate 101 and the shape of the second substrate 201 are both rectangular. In some examples, the size of the first substrate 101 is 3.2 cm*4.5 cm, and the size of the second substrate 201 is 3.2 cm*3 cm. In some embodiments, the size of the second substrate 201 is smaller than the size of the first substrate 101, the second substrate 201 covers the reaction area 1001, and the orthographic projection of the second substrate 201 on the first substrate 101 can completely overlap with the reaction area 1001. It should be noted that the embodiments of the present disclosure are not limited to this. In some other examples, the size of the second substrate 201 may also be the same as the size of the first substrate 101. In this case, the second substrate 201 covers the reaction area 1001 and the peripheral area 1002. For example, the orthographic projection of the second substrate 201 on the first substrate 101 may completely coincide with the first substrate 101.

The second hydrophobic layer 202 has hydrophobic and lipophilic characteristics, and is located on a side of the second substrate 201 facing the first substrate 101. By providing the second hydrophobic layer 202, the reaction system solution can more easily enter each recess 103. In an example, the material of the second hydrophobic layer 202 comprises SiNₓ. In an alternative example, the second hydrophobic layer 202 is a light-absorbing layer, and the material of the light-absorbing layer comprises at least one of TiO₂ and TiON. By designing the second hydrophobic layer 202 as a light-absorbing layer, some unused excitation light can be further absorbed and hence can be prevented from being irradiated on the first substrate 101 and/or the defining layer 102 in a certain way, thereby the fluorescence interference of the first substrate 101 and/or the defining layer 102 can be further reduced.

The counter substrate 2000 comprises at least one through hole penetrating the second substrate 201 and the second hydrophobic layer 202. As illustrated in the figure, the counter substrate 2000 comprises a sample inlet hole 203 and a sample outlet hole 205, and both the sample inlet hole 203 and the sample outlet hole 205 penetrate the second substrate 201 and the second hydrophobic layer 202. In an example, the reaction system solution can be injected into the sample inlet hole 203 through a micro-syringe pump or through a pipette, and then into each recess 103 through a self-aspiration liquid manner. In some embodiments, with reference to FIG. 5, the reaction area 1001 further comprises a non-functional area 1001b. The sample inlet hole 203 and the sample outlet hole 205 are both located in the non-functional area 1001b, and are located on different sides of the functional area 1001a, such as symmetrically distributed on different sides of the functional area 1001a. As illustrated in FIG. 5, in the first direction X, the sample inlet hole 203 and the sample outlet hole 205 are respectively located on both sides of the functional area 1001a. For example, the sample inlet hole 203 and the sample outlet hole 205 are symmetrically distributed with respect to the second direction Y, so that the flow of the reaction system solution in the microfluidic device 500 can be more uniform, and the reaction system solution can easily enter each recess 103. Of course, the embodiment of the present disclosure is not limited to this, and the sample inlet hole 203 and the sample outlet hole 205 may also be symmetrically distributed with respect to the first direction X or other arbitrary directions. It should be noted that the sample inlet hole 203 and the sample outlet hole 205 may also be located in the functional area 1001a.

Continuing to refer to FIG. 10, the microfluidic device 500 further comprises a plurality of sealants 204. The plurality of sealants 204 are arranged in the peripheral area 1002 and located between the array substrate 100 and the counter substrate 2000. The plurality of sealants 204 are configured to maintain the space between the array substrate 100 and the counter substrate 2000, thereby providing a space for the flow of the reaction system solution. In some embodiments, some sealants 204 may also be arranged in the reaction area 1001, for example, dispersedly arranged in multiple places in the reaction area 1001, so as to improve the compressive strength of the microfluidic device 500 and prevent the microfluidic device 500 from being damaged due to external force applied to the reaction area 1001. In some embodiments, the size and shape of the plurality of sealants 204 may be the same as each other, thereby improving the thickness uniformity of the microfluidic device 500. In an alternative embodiment, the size and shape of the plurality of sealants 204 can also be set according to the possible stress conditions of the microfluidic device 500. For example, the size of the sealant 204 is larger on the periphery and the center of the microfluidic device 500, while the size of the sealant 204 is smaller in the remaining positions of the microfluidic device 500.

In some embodiments, in a direction perpendicular to the first substrate 101, the height of the sealant 204 is greater than the height of the defining layer 102, the first substrate 101, the defining layer 102, and the sealant 204 jointly define the sample injection channel and the sample output channel of the reaction system solution to ensure that the reaction system solution can move into each recess 103, and the reaction system solution that does not enter the recess 103 flows out of the space between the array substrate 100 and the counter substrate 2000. In some embodiments, the height of the sealant 204 is 30% or 50% larger than the height of the defining layer 102, and the specific ratio between the two may be determined according to actual requirements, which is not limited in the embodiments of the present disclosure.

The material of the sealant 204 may be a curable organic material, such as a thermal curing material or a light curing material, for example, an ultraviolet (UV) hardening acrylic resin or other suitable materials. The shape of the sealant 204 may be a spherical shape. In this case, the array substrate 100 and the counter substrate 2000 can be cured and packaged by the sealant 204, so that the array substrate 100 and the counter substrate 2000 can be assembled together. In this way, the sealant 204 can control the distance between the array substrate 100 and the counter substrate 2000. The embodiments of the present disclosure include but are not limited thereto. The shape of the sealant 204 can also be any suitable shape such as a columnar shape or an ellipsoidal shape.

In some embodiments, the microfluidic device 500 may further comprise a first temperature sensor (not illustrated in the figure). The first temperature sensor is arranged on a side of the first substrate 101 away from the second substrate 201 and is located in the reaction area 1001. The first temperature sensor is configured to detect the temperature of the reaction area 1001. For example, the temperature of the reaction area 1001 needs to be maintained at a predetermined temperature (for example, 95°C, 55°C, or 72°C, etc.). The first temperature sensor can detect the temperature of the reaction area 1001 in real time, and then adjust the temperature of the reaction area 1001 in real time through the heating electrode 106 to keep the temperature of the reaction area 1001 at a predetermined temperature, thereby preventing the temperature of the reaction area 1001 from being too high or too low to affect the amplification reaction. The first temperature sensor may be various types of temperature sensors, comprising but not limited to a contact temperature sensor or a non-contact temperature sensor, such as a thermocouple temperature sensor or an infrared temperature sensor.

The microfluidic device 500 provided by the embodiments of the present disclosure can have basically the same technical effects as the array substrates described in the previous embodiments. Therefore, for the sake of brevity, the description will not be repeated herein.

According to still another aspect of the present disclosure, a microfluidic system is provided, which comprises a control device and the microfluidic device 500 described in any of the foregoing embodiments. The control device is electrically connected to the microfluidic device 500 and is configured to control the temperature of the microfluidic device 500. The microfluidic system helps to make the droplets automatically enter the recesses 103 of the microfluidic device 500, can achieve effective sample injection and avoid cross-flow, and can effectively realize the temperature control of the recesses 103 of the microfluidic device 500. The temperature cycle can be realized without driving the liquid droplets, and no external heating equipment is needed. The integration is high, the operation is simple, and the production cost is low.

Fig. 11 illustrates a schematic block diagram of a microfluidic system 600 according to an embodiment of the present disclosure. As illustrated in FIG. 11, the microfluidic system 600 comprises a microfluidic device 500, a control device 620, and a power supply device 630. The power supply device 630 provides a signal voltage or a driving voltage or the like to the microfluidic device 500 and the control device 620. The control device 620 is electrically connected to the microfluidic device 500 and is configured to apply an electrical signal to the microfluidic device 500 to drive the heating electrode 106 of the microfluidic device 500. The multiple recesses 103 of the microfluidic device 500 can accommodate the reaction system solution. The control device 620 applies an electrical signal to the heating electrode 106 of the microfluidic device 500 to cause the heating electrode 106 to release heat, thereby controlling the temperature of the functional area of the microfluidic device 500, so that the reaction system solution undergoes the amplification reaction. For example, the control device 620 may be implemented as general-purpose or special-purpose hardware, software, or firmware, etc., for example, it may also comprise a central processing unit (CPU), an embedded processor, a programmable logic controller (PLC), etc. The embodiment of the present disclosure does not limit this.

In some embodiments, the microfluidic system 600 may optionally further comprise a second temperature sensor 650. For example, when the microfluidic device 500 does not comprise the first temperature sensor, the second temperature sensor 650 needs to be provided in the microfluidic system 600, and the second temperature sensor 650 needs to be installed in the microfluidic device 500 at a position basically the same as that of the first temperature sensor, so as to realize the function of detecting temperature. For example, the second temperature sensor 650 is arranged on the side of the first substrate 101 of the microfluidic device 500 away from the second substrate 201 and is located in the reaction area 1001 of the array substrate 100, and the second temperature sensor 650 is configured to detect the temperature of the reaction area 1001 of the microfluidic device 500. The second temperature sensor 650 may be various types of temperature sensors, comprising but not limited to a contact temperature sensor or a non-contact temperature sensor, such as a thermocouple temperature sensor or an infrared temperature sensor. It should be noted that in some other embodiments, when the microfluidic device 500 comprises the first temperature sensor, the microfluidic system 600 comprising the microfluidic device 500 does not need to provide the second temperature sensor 650.

The microfluidic system 600 may further comprise an optical unit 640 configured to perform optical detection on the microfluidic device 500. In some embodiments, the optical unit 640 comprises a fluorescence detection device configured to perform fluorescence detection on the reagent to be tested in the plurality of recesses 103. For example, the fluorescence detection device may comprise a fluorescence light source and an image sensor (for example, a charge coupled device (CCD) image sensor). The optical unit 640 may, for example, further comprise an image processing device configured to process the detection image output by the fluorescence detection device. For example, the image processing device may comprise a central processing unit (CPU) or a graphics processing unit (GPU) or the like. For example, the control device 620 is also configured to control the fluorescence detection device and the image processing device to perform corresponding functions.

The working principle and process of the microfluidic system 600 are described as follows.

First, prepare the reaction system solution. For example, the reaction system solution may comprise a cell lysis solution, a DNA fragment sample solution fragmented by a DNA lyase, and PCR amplification reagents. In an example, it is assumed that the DNA to be tested is exon 19 of the epidermal growth factor receptor (EGFR) gene, and correspondingly, the PCR amplification reagents comprise PCR amplification primers specific for exon 19 of the EGFR gene. For example, the volume of the reaction system solution is 20 microliters, and the reaction system solution comprises 10 microliters of MIX reagent (MIX reagents comprise Taq enzyme, dNPTs and MgCl₂), 0.6 microliters of upstream primers (10 millimoles (mM)), 0.6 microliters (10mM) of downstream primers, 7.8 microliters of water and 1 microliter of fully diluted template deoxyribonucleic acid (DNA), to ensure that the amount of template DNA in each micro-reaction chamber is less than or equal to 1.

Then, install a polytetrafluoroethylene joint and a silicone tube into the sample inlet hole 203 of the microfluidic device 500, and inject the prepared reaction system solution into the sample inlet hole 203 through a micro-syringe pump or a pipette. The reaction system solution enters the sample inlet hole 203 through the polytetrafluoroethylene joint and the silicone tube, and then the reaction system solution enters each recess 103 through self-absorption under the cooperation of the hydrophilic layer 108 and the first hydrophobic layer 109.

Next, a three-step method dPCR is used to carry out the thermal cycle amplification process. The oil-sealed microfluidic device 500 is placed on the chip carrier of the microfluidic system 600 and fixed by a clamp, so that the conductive layer 107 of the microfluidic device 500 and an electrode are electrically connected. For example, the parameter setting button is used to set the parameters. The cycle parameters are denaturation at 95°C for 15 seconds, annealing at 55°C for 45 seconds, and extension at 72°C for 45 seconds. A total of 30 thermal cycles are set. For example, the pre-denaturation at 95°C for 5 minutes can also be set. The droplets in the micro-reaction chamber containing template DNA in the microfluidic device 500 undergo PCR amplification reaction, while the droplets in the micro-reaction chamber without template DNA serve as a control group.

It should be noted that before PCR amplification, the recess 103 can be filled with a bovine serum albumin (BSA) solution of 0.2% mass fraction and soaked for 1 hour to reduce the adsorption of the PCR reagents and the sample template by the inner surface of the recess 103, and improve the reaction efficiency and detection accuracy. Then, the BSA solution is pumped clean with a micro-pump, the reaction system solution is injected into the recess 103, and then seal the device with oil phase liquid. The material of the oil phase liquid may be mineral oil, liquid paraffin isopropyl palmitate, butyl laurate, perfluoroalkane oil, etc., and the oil phase liquid may be used to seal the sample inlet hole 203 and the sample outlet hole 205 to prevent the reaction system solution from volatilizing.

After 30 cycles of amplification, the reaction system solution in the recess 103 becomes the reagent to be tested after undergoing polymerase chain reaction. The microfluidic device 500 is taken out, and the microfluidic device 500 is observed by a fluorescence microscope. The excitation wavelength may be, for example, 450 nm to 480 nm, so as to obtain a fluorescence spectrum. In an example, when the reagent to be tested contains exon 19 of the EGFR gene mutation, since the reagent to be tested comprises the specific PCR amplification primers of exon 19 of the EGFR gene mutation, under the action of the PCR amplification primers, the mutated exon 19 is greatly amplified, so that the reagent to be tested shows a positive result, that is, at least a part of the reagent to be tested undergoes a fluorescence reaction. In another example, when the reagent to be tested does not contain exon 19 of the EGFR gene mutation, the reagent to be tested shows a negative result, that is, the reagent to be tested does not have a fluorescence reaction. As a result, the detection of exon 19 of the EGFR gene can be achieved.

The microfluidic system 600 provided by the embodiments of the present disclosure may have basically the same technical effects as the array substrates described in the previous embodiments. Therefore, for the sake of brevity, the description will not be repeated herein.

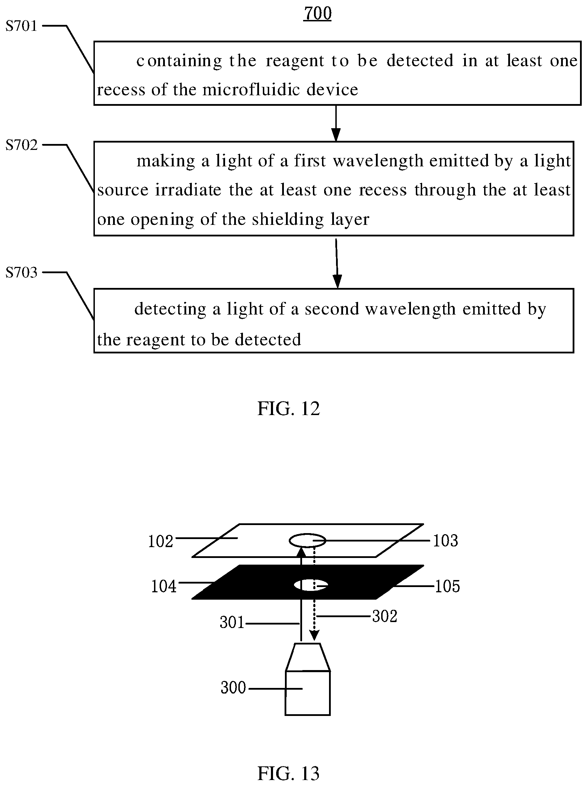

According to yet another aspect of the present disclosure, a fluorescence detection method is provided. FIG. 12 illustrates a flowchart of a fluorescence detection method 700 according to an embodiment of the present disclosure, and FIG. 13 illustrates a schematic diagram of a fluorescence detection process of a microfluidic device 500 according to an embodiment of the present disclosure. The fluorescence detection method 700 will be described below in conjunction with FIG. 12 and FIG. 13.

S701: containing the reagent to be tested in at least one recess of the microfluidic device.

In an example, the prepared reaction system solution is injected into the sample inlet hole 203 of the microfluidic device 500, and the reaction system solution enters each recess 103 of the microfluidic device 500 through self-absorption under the cooperation of the hydrophilic layer 108 and the first hydrophobic layer 109. After the reaction system solution in each recess 103 undergoes the polymerase chain reaction, that is, after the amplification reaction is completed, the aforementioned reagent to be tested is formed.

S702: making a light of a first wavelength emitted by the light source irradiate the at least one recess through the at least one opening of the shielding layer.

As illustrated in FIG. 13, the fluorescence detection device 300 comprises a light source system (not illustrated) that emits light 301 of a first wavelength. The light 301 of the first wavelength is irradiated to the corresponding plurality of recesses 103 defined by the defining layer 102 through the plurality of openings 105 of the shielding layer 104, thereby exciting the reagent to be tested in each recess 103 to emit fluorescence. The light 301 of the first wavelength illustrated in the figure is incident from the bottom to the top, because the shielding layer 104 is located below the defining layer 102. When the shielding layer 104 is located above the defining layer 102 (as illustrated in FIGS. 8 and 9), the light 301 of the first wavelength can also be incident from the top to the bottom. In addition, it should be noted that the light source system may be integrated in the fluorescence detection device 300 or independent of the fluorescence detection device 300, which is not limited in the embodiments of the present disclosure.

S703: detecting a light of a second wavelength emitted by the reagent to be tested.

In an example, after the reagent to be tested in the recess 103 is excited by the light 301 of the first wavelength, the light 302 of the second wavelength is emitted by the reagent to be tested, and the second wavelength is greater than the first wavelength. For example, the light 301 of the first wavelength may be blue light, and the light 302 of the second wavelength may be green light; alternatively, the light 301 of the first wavelength may be yellow light, and the light 302 of the second wavelength may be red light. The fluorescence detection device 300 may comprise, for example, a fluorescence light source and an image sensor (for example, a charge coupled device (CCD) image sensor). The image processing device is configured to process the detection picture output by the fluorescence detection device 300. For example, the image processing device may comprise a central processing unit (CPU) or a graphics processing unit (GPU) or the like.

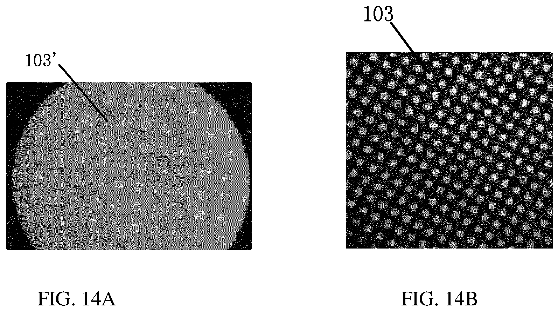

FIG. 14A illustrates a fluorescence picture of a conventional microfluidic device that does not comprise a shielding layer, and FIG. 14B illustrates a fluorescence picture of a microfluidic device 500 according to an embodiment of the present disclosure. Each dot in FIG. 14A represents the recess 103' of the microfluidic device. It can be seen from FIG. 14A that each recess 103' and its surrounding area present substantially the same color. This is because the conventional microfluidic device is not provided with a shielding layer. When the excitation light irradiates the recess, not only the reagent to be tested in the recess is excited to emit fluorescence, but also the substrate and defining layer in the microfluidic device are excited to emit fluorescence. Compared with the usually small volume of the reagent to be tested, the background fluorescence caused by the substrate and the defining layer in the microfluidic device greatly affects the fluorescence detection accuracy of the reagent to be tested in the recess, making it impossible to obtain accurate detection results. Therefore, there is a phenomenon that almost all areas emit fluorescence as illustrated in FIG. 14A.

Each dot in FIG. 14B represents the recess 103 of the microfluidic device 500 according to an embodiment of the present disclosure. It can be seen from FIG. 14B that the color presented by each recess 103 and the color presented by the surrounding area of each recess 103 are very different, and the contrast is very obvious. Each recess 103 presents a relatively bright color, and the surrounding area presents black color. This is because the shielding layer 104 is provided in the microfluidic device 500, and the orthographic projection of the shielding layer 104 on the first substrate 101 at least partially overlaps the orthographic projection of the defining layer 102 on the first substrate 101. That is to say, the shielding layer 104 shields at least a part or all of the defining layer 102, and the orthographic projection of the shielding layer 104 on the first substrate 101 necessarily also at least partially overlaps the first substrate 101 itself. When the light 301 of the first wavelength is irradiated to the recess 103 through the opening 105 of the shielding layer 104, the shielding layer 104 can shield the first substrate 101 and the defining layer 102. Therefore, the shielding layer 104 can not only prevent the defining layer 102 from being irradiated by the light 301 of the first wavelength, but can also block the fluorescence emitted by the first substrate 101 irradiated by the light 301 of the first wavelength from transmitting through the shielding layer 104.Therefore, the light 301 of the first wavelength can only be irradiated to the recess 103 through the opening 105 to excite the reagent to be tested in the recess 103 to emit fluorescence, so that the position corresponding to the recess 103 in FIG. 14B presents a relatively bright color, while the area other than the recess 103 appears black. Therefore, through such a fluorescence detection method, the fluorescence interference caused by the first substrate 101 and the defining layer 102 can be reduced or even avoided, so that the fluorescence signal emitted by the reagent to be tested in the recess 103 can be accurately identified by the detector, so that it can be more sensitively and accurately to read the reaction signal, the fluorescence detection accuracy of the reagent to be tested is improved, and image data support for subsequent nucleic acid amplification reaction data analysis is provided. In addition, through this detection method, clearer micro-hole array imaging can be achieved, detection errors caused by false positives can be reduced, and interference between different channels in the process of multi-channel fluorescence signal detection can be well avoided.

Another aspect of the present disclosure provides a method 800 for manufacturing a microfluidic device 500, which may comprise the array substrate described in any of the previous embodiments. Hereinafter, the method steps are briefly described by taking the microfluidic device 500 comprising the array substrate 100 as an example.

Step 801: providing a first substrate 101. The first substrate 101 may be made of any suitable material. In an example, the first substrate 101 is made of glass.

Step 802: forming a conductive film layer on the first substrate 101 at about 240°C. In an example, a molybdenum (Mo) layer with a thickness of 200 Å, an aluminum neodymium (AlNd) layer with a thickness of 3000 Å, and a molybdenum (Mo) layer with a thickness of 800 Å are sequentially deposited on the first substrate 101 to form a conductive film layer. The conductive film layer is patterned, such as exposure, development, etching, etc., to form a conductive layer 107.

Step 803: depositing a first insulating film layer on the conductive layer 107 at about 200° C, and patterning the first insulating film layer to form a first insulating layer 110 covering the conductive layer 107. In an example, the first insulating layer 110 is a SiO₂ layer with a thickness of about 3000 Å.

Step 804: patterning the first insulating layer 110 to form at least one via 112 penetrating the first insulating layer 110, and the at least one via 112 exposes a part of the conductive layer 107. In an example, the first insulating layer 110 is etched in a dry etching machine to form the via 112. The specific process is described as follows: etching for 10s at a pressure of about 150 mtorr, a power of about 800 W, and the volumetric flow rate of O₂ of 400 sccm (standard cubic centimeter per minute); etching for 200s at a pressure of about 60 mtorr, a power of about 800 W, and a gas volume flow ratio of CF₄ and O₂ of about 200:50; etching for 30s at a pressure of about 130 mtorr, a power of about 800 W, and a gas volume flow ratio of O₂ and CF₄ of about 400:40; and etching for 160s at a pressure of about 60 mtorr, a power of about 800 W, and a gas volume flow ratio of CF₄ and O₂ about 200:50.

Step 805: depositing a conductive film layer on a side of the first insulating layer 110 away from the first substrate 101, and then performing processes such as exposure, development, etching, and peeling of the conductive film layer to form a patterned heating electrode 106. In an example, the material of the heating electrode 106 is ITO. In an example, the heating electrode 106 comprises a plurality of sub-parts separated from each other.

Step 806: depositing a second insulating film layer on a side of the heating electrode 106 away from the first substrate 101, and patterning the second insulating film layer to form a second insulating layer 111 that at least partially covers the heating electrode 106. In an example, the material of the second insulating layer 111 is SiO₂. In another example, the second insulating layer 111 comprises a SiO₂ layer with a thickness of about 1000 Å and a SiNₓ layer with a thickness of about 2000 Å that are sequentially stacked.

Step 807: coating a shielding film layer on a side of the second insulating layer 111 away from the first substrate 101, and patterning the shielding film layer to form a shielding layer 104 that defines openings 105. In an example, the specific steps of forming the shielding layer 104 may comprise: spin-coating the shielding film layer on the side of the second insulating layer 111 away from the first substrate 101 under the condition of a pressure of 30 Kpa, and the spin coating speed is about 380 r/min, spin coating time is about 7 seconds. Then, the spin-coated shielding film layer is pre-cured at 90°C for 120 seconds. Then, the shielding film layer is exposed, developed, and etched through a mask, and the development time is about 75 seconds. Finally, the etched shielding film layer is post-cured at 230° C for about 20 minutes to form the shielding layer 104 defining the openings 105. The thickness of the shielding layer 104 is, for example, in the range of 0.6-2.4 microns, for example, 2 microns. In an example, the material forming the shielding layer 104 comprises chromium, chromium oxide, and black resin.

Step 808: coating a defining film layer on a side of the shielding layer 104 away from the first substrate 101, and patterning the defining film layer to form a defining layer 102 that defines a plurality of recesses 103. In an example, the process of forming the defining layer 102 is described as follows: first, under a pressure of 30 Kpa, the optical glue is spin-coated on the surface of the shielding layer 104 away from the first substrate 101 at a speed of 300 r/min. The spin-coating time is about 10 seconds. Then the optical glue is cured for 120 seconds at a temperature of 90°C. Repeat the above process twice to obtain a defining film layer. Next, the defining film layer is exposed through a mask, and then the exposed defining film layer is developed with a developing solution for 100 seconds, and then etched. At a temperature of 230° C, the etched defining film layer is cured for 30 minutes, and finally a defining layer 102 defining a plurality of recesses 103 is obtained. The material of the defining layer 102 comprises photoresist. The orthographic projection of each opening 105 of the shielding layer 104 on the first substrate 101 at least partially overlaps the orthographic projection of a corresponding recess 103 of the defining layer 102 on the first substrate 101, and the orthographic projection of the shielding layer 104 on the first substrate 101 at least partially overlaps the orthographic projection of the defining layer 102 on the first substrate 101. In an example, the recess 103 of the defining layer 102 is a cylinder, the bottom diameter of the recess 103 is 50 microns, the depth is between 40 and 50 microns, and the distance between the centers of two adjacent recesses 103 is 100 microns.

Step 809: at 200° C, depositing an insulating film layer on the surface of the defining layer 102 away from the first substrate 101, and exposing, developing, and etching the insulating film layer to form a patterned layer. The patterned layer is treated with a 0.4% KOH solution for about 15 minutes to perform hydrophilic modification on the patterned layer, thereby forming the hydrophilic layer 108. The hydrophilic layer 108 covers the surface of the defining layer 102 away from the first substrate 101 and at least covers the sidewall of each recess 103. In an example, the hydrophilic layer 108 is a SiO₂ layer with a thickness of about 3000 Å.

Step 810: depositing an insulating film layer on the surface of the hydrophilic layer 108 away from the first substrate 101, and exposing, developing, and etching the insulating film layer to form the first hydrophobic layer 109. In an example, the process of forming the first hydrophobic layer 109 is as follows: in a plasma enhanced chemical vapor deposition (PECVD) equipment, at a temperature of about 200°C, a power of about 600 W, a pressure of about 1200 mtorr, and the distance between the plasma reaction enhancement target and the sample to be deposited in the PECVD equipment of about 1000 mils, SiH₄ (volumetric flow rate of 110 sccm), NH₃ (volumetric flow rate of 700 sccm), and N₂ (volumetric flow rate of 2260 sccm, access time of 100 seconds) are passed into the reaction chamber to deposit a SiNₓ film with a thickness of 1000 Å on the surface of the hydrophilic layer 108 away from the first substrate 101. The SiNₓ film layer is exposed, developed, and etched to form the first hydrophobic layer 109.

Step 811: packaging the array substrate 100 on which the hydrophilic treatment and hydrophobic treatment have been completed.

Step 812: providing a second substrate 201. The second substrate 201 may be made of any suitable material. In an example, the second substrate 201 is made of glass.

Step 813: depositing a film on a side of the second substrate 201 close to the first substrate 101, and processing the film to form a second hydrophobic layer 202, which is a TiO₂ layer with a thickness of about 1000 Å. In an example, the second hydrophobic layer 202 is made of a light-absorbing material, and the light-absorbing material comprises at least one of TiO₂ and TiON. In another example, the second hydrophobic layer 202 is formed of SiNₓ. The second substrate 201 and the second hydrophobic layer 202 constitute a counter substrate 2000 opposite to the array substrate 100.

Step 814: perforating the second substrate 201 and the second hydrophobic layer 202 to form at least one sample inlet hole 203 and at least one sample outlet hole 205 penetrating the second substrate 201 and the second hydrophobic layer 202. In an example, the diameter of the at least one sample inlet hole 203 and the at least one sample outlet hole 205 is between 0.6 mm and 1.2 mm.

Step 815: curing and assembling the array substrate 100 and the counter substrate 2000 with a sealant, and defining the space between the array substrate 100 and the counter substrate 2000.

It should be noted that the manufacturing method may further comprise more steps, which may be determined according to actual requirements, which are not limited in the embodiments of the present disclosure. For the technical effects achieved by the manufacturing method, reference may be made to the above description of the array substrate 100 and the microfluidic device 500, which will not be repeated herein.

When the microfluidic device 500 comprises the array substrate 200 illustrated in FIG. 7, the manufacturing method of the microfluidic device 500 is basically the same as the method 800 described above, except that the sequence of steps is slightly different. According to the above steps 801-806 and steps 808-811, the first substrate 101, the conductive layer 107, the first insulating layer 110, the heating electrode 106, the second insulating layer 111, the defining layer 102, the recess 103, the hydrophilic layer 108 and the first hydrophobic layer 109 are sequentially prepared and packaged, that is, step 807 is omitted (i.e., the step of preparing the shielding layer 104 is omitted). After the packaging is completed, the array substrate formed with the above-mentioned film layers is turned over, a shielding film layer is coated on the side of the first substrate 101 away from the defining layer 102, and the shielding film layer is patterned to form a shielding layer 104 defining openings 105. In an example, the specific steps of forming the shielding layer 104 may comprise: spin-coating a shielding film layer on the side of first substrate 101 away from the defining layer 102 under the condition of a pressure of 30 Kpa, and the spin coating speed is about 380 r/min, spin coating time is about 7 seconds. Then, the spin-coated shielding film layer is pre-cured at 90°C for 120 seconds. Then, the shielding film layer is exposed, developed, and etched through a mask, and the development time is about 75 seconds. Finally, the etched shielding film layer is post-cured at 230° C for about 20 minutes to form the shielding layer 104 defining the openings 105. The orthographic projection of each opening 105 of the shielding layer 104 on the first substrate 101 at least partially overlaps the orthographic projection of a corresponding recess 103 of the defining layer 102 on the first substrate 101, and the orthographic projection of the shielding layer 104 on the first substrate 101 at least partially overlaps the orthographic projection of the defining layer 102 on the first substrate 101. The thickness of the shielding layer 104 is, for example, in the range of 0.6-2.4 microns, for example, 2 microns. In an example, the material forming the shielding layer 104 comprises chromium, chromium oxide, and black resin.

Then, at about 200° C., a third insulating film layer is deposited on the side of the shielding layer 104 away from the first substrate 101, and the third insulating film layer is patterned to form a third insulating layer 115. The third insulating layer 115 has a protective effect on the shielding layer 104. In an example, the third insulating layer 115 is SiO₂ with a thickness of about 3000 Å.

Finally, continue to prepare the counter substrate 2000 according to the above steps 812-815, and perform curing and packaging on the array substrate 200 and the counter substrate 2000, so that the microfluidic device 500 comprising the array substrate 200 is prepared.

When the microfluidic device 500 comprises the array substrate 300 illustrated in FIG. 8, the manufacturing method of the microfluidic device 500 is basically the same as the method 800 described above, except that the order of step 807 and step 808 is reversed. That is, after the first substrate 101, the conductive layer 107, the first insulating layer 110, the heating electrode 106, and the second insulating layer 111 are sequentially prepared in the order of the above steps 801-806, prepare a defining layer 102 defining a plurality of recesses 103 on the surface of the second insulating layer 111 away from the first substrate 101 according to step 808. Then, the shielding layer 104 defining the openings 105 is prepared on the surface of the defining layer 102 away from the first substrate 101, and the preparation process is the same as that described in the foregoing step 807. The formed shielding layer 104 covers the side surfaces of the defining layer 102 and the surface of the defining layer 102 away from the first substrate 101. Finally, continue the subsequent preparation in line with steps 809 to 815 to complete the microfluidic device 500 comprising the array substrate 300.

When the microfluidic device 500 comprises the array substrate 400 illustrated in FIG. 9, the manufacturing method of the microfluidic device 500 is basically the same as the method 800 described above, except that another step is added between step 808 and step 809. That is, the first substrate 101, the conductive layer 107, the first insulating layer 110, the heating electrode 106, the second insulating layer 111, the shielding layer 104 and the defining layer 102 are sequentially prepared according to the above steps 801-808. In step 808, the process of preparing an example of the defining layer 102 is as follows: first, under a pressure of 30 Kpa, an optical glue is spin-coated on the surface of the shielding layer 104 away from the first substrate 101 at a speed of 200 r/min, and the spin coating time is about 10 seconds, then, cure the optical glue for 120 seconds at a temperature of 90°C. Next, the optical glue is exposed through a mask, and then the exposed optical glue is developed with a developing solution for 240 seconds and etched. At a temperature of 230° C., the etched optical glue is cured for 30 minutes, and finally a defining layer 102 that defines a plurality of recesses 103 is obtained. The material of the defining layer 102 comprises photoresist.

After the defining layer 102 is prepared, the shielding layer 104 defining the openings 105 is again prepared on the surface of the defining layer 102 away from the first substrate 101, and the preparation method is the same as that described in step 807. The formed shielding layer 104 covers the side surfaces of the defining layer 102 and the surface of the defining layer 102 away from the first substrate 101. Finally, follow steps 809-815 to continue to complete subsequent preparations, so as to obtain the microfluidic device 500 comprising the array substrate 400. That is, when preparing the microfluidic device 500 comprising the array substrate 400, the shielding layer 104 needs to be prepared twice before and after the process of preparing the defining layer 102.

Another aspect of the present disclosure provides a method 900 for using the microfluidic device 500. The method 900 may comprise the following steps:
Step 901: making the reaction system solution enter a plurality of recesses 103 of the microfluidic device 500 through the sample inlet hole 203 of the microfluidic device 500;
Step 902: supplying an electrical signal to the conductive layer 107 of the microfluidic device 500, so as to drive the heating electrode 106 through the conductive layer 107 to heat the plurality of recesses 103.

In some embodiments, the method 900 further comprises: cooling the plurality of recesses 103 to change the temperature of the plurality of recesses 103, so that the reaction system solution in the plurality of recesses 103 undergoes a temperature cycle of a denaturation stage, an annealing stage, and an extension stage. For example, it can be cooled by air-cooled equipment, which has a simple structure and is easy to implement.

It should be noted that the method 900 may further comprise more steps, which may be determined according to actual requirements, and the embodiment of the present disclosure does not limit this.

In the description of this specification, the description with reference to the terms "one embodiment", "another embodiment", etc., means that specific features, structures, materials, or characteristics described in conjunction with the embodiment is comprised in at least one embodiment of the present disclosure.

Unless otherwise defined, the technical or scientific terms used in the present disclosure shall have the usual meanings understood by those with ordinary skills in the field to which this disclosure belongs. The "first", "second" and similar words used in the present disclosure do not indicate any order, quantity or importance, but are only used to distinguish different components. "comprise" or "include" and other similar words mean that the element or item appearing before the word encompasses the element or item listed after the word and its equivalents, but does not exclude other elements or items. Similar words such as "connect" or "connection" are not limited to physical or mechanical connections, but may include electrical connections, whether direct or indirect. "Up", "down", "left", "right", etc., are only used to indicate the relative position relationship. When the absolute position of the described object changes, the relative position relationship may also change accordingly.

As those skilled in the art will understand, although the various steps of the method in the present disclosure are described in a specific order in the drawings, this does not require or imply that these steps must be performed in the specific order, unless the context clearly indicates otherwise. Additionally or alternatively, multiple steps can be combined into one step for execution, and/or one step can be decomposed into multiple steps for execution. In addition, other method steps can be inserted between the steps. The inserted steps may represent such as an improvement of the method described herein, or may be unrelated to the method. In addition, a given step may not be fully completed before the next step starts.

The above are only specific implementations of the present disclosure, but the protection scope of the present disclosure is not limited thereto. Therefore, the protection scope of the present disclosure is defined by the protection scope of the claims.

## Claims

1. An array substrate, comprising a plurality of recesses (103) arranged in an array, wherein the array substrate is in a plane, and a ratio of an area of an orthographic projection of the plurality of recesses (103) on the plane to an area of an orthographic projection of the array substrate on the plane is between 0.05 and 0.60,
wherein the array substrate further comprises:
a first substrate (101);
a defining layer (102) on the first substrate (101) and defining the plurality of recesses (103);
a shielding layer (104) defining a plurality of openings (105), an orthographic projection of each of the plurality of openings (105) on the first substrate (101) at least partially overlapping an orthographic projection of a respective one of the plurality of recesses (103) on the first substrate (101), and an orthographic projection of the shielding layer (104) on the first substrate (101) at least partially overlapping an orthographic projection of the defining layer (102) on the first substrate (101);
a hydrophilic layer (108); and
a first hydrophobic layer (109),
wherein the hydrophilic layer (108) covers a sidewall and a bottom of each of the plurality of recesses (103) as well as a surface of the defining layer (102) away from the first substrate (101), the first hydrophobic layer (109) is on a side of the defining layer (102) away from the first substrate (101) and farther away from the first substrate (101) than the hydrophilic layer (108);
**characterized in that**,
the first hydrophobic layer (109) defines a plurality of holes (114), the plurality of holes (114), the plurality of recesses (103), and the plurality of openings (105) correspond one by one with each other;
both a first orthographic projection of each of the plurality of holes (114) on the first substrate (101) and a third orthographic projection of an opening (105) corresponding to the hole (114) on the first substrate (101) are within a second orthographic projection of a recess (103) corresponding to the hole (114) on the first substrate (101), and the first orthographic projection, the second orthographic projection, and the third orthographic projection form concentric rings; and
the first orthographic projection is between the second orthographic projection and the third orthographic projection, and the third orthographic projection is within the first orthographic projection.

2. The array substrate according to claim 1, wherein the plurality of recesses (103) penetrate the defining layer (102).

3. The array substrate according to claim 1 or 2, wherein
the shielding layer (104) is between the first substrate (101) and the defining layer (102), or
the shielding layer (104) is on a side of the first substrate (101) away from the defining layer (102).

4. The array substrate according to claim 1 or 2,
wherein the shielding layer (104) comprises a first portion (104a), the first portion (104a) is on a side of the defining layer (102) away from the first substrate (101), and the first portion (104a) is attached to side surfaces of the defining layer (102) and a surface of the defining layer (102) away from the first substrate (101); and
wherein the first portion (104a) defines the plurality of openings (105).

5. The array substrate according to claim 4,
wherein the shielding layer (104) further comprises a second portion (104b), the second portion (104b) is attached to a surface of the defining layer (102) close to the first substrate (101) to surround the defining layer (102) together with the first portion (104a);
wherein the second portion (104b) defines the plurality of openings (105); and
wherein an orthographic projection of the plurality of openings (105) defined by the first portion (104a) of the shielding layer (104) on the first substrate (101) and an orthographic projection of the plurality of openings (105) defined by the second portion (104b) of the shielding layer (104) on the first substrate (101) completely overlap.

6. The array substrate according to any one of claims 1-2 and 4-5, wherein a surface of the defining layer (102) close to the first substrate (101) and/or a surface of the defining layer (102) away from the first substrate (101) constitute the shielding layer (104).

7. The array substrate according to any one of claims 1-5,
wherein the plurality of recesses (103) correspond to the plurality of openings (105) one by one, and an orthographic projection of each of the plurality of openings (105) on the first substrate (101) is within an orthographic projection of a recess (103) corresponding to the opening (105) on the first substrate (101).

8. The array substrate according to any one of claims 1-5, wherein both a shape of an orthographic projection of each of the plurality of recesses (103) and a shape of an orthographic projection of each of the plurality of openings (105) on the first substrate (101) comprise a circle or a regular polygon,
wherein the plurality of recesses (103) correspond to the plurality of openings (105) one by one, and an orthographic projection of each of the plurality of openings (105) on the first substrate (101) is within an orthographic projection of a recess (103) corresponding to the opening (105) on the first substrate (101); and
wherein both a shape of an orthographic projection of each opening (105) and a shape of an orthographic projection of a recess (103) corresponding to the opening (105) on the first substrate (101) are circular, a diameter of each opening (105) is in a range of 20-80 µm, and a diameter of the recess (103) corresponding to the opening (105) is in a range of 25-90 µm; or a shape of an orthographic projection of each opening (105) on the first substrate (101) is a first regular polygon, and a shape of an orthographic projection of the recess (103) corresponding to the opening (105) on the first substrate (101) is a second regular polygon, a diameter of an inscribed circle of the first regular polygon is in a range of 20-80 µm, and a diameter of an inscribed circle of the second regular polygon is in a range of 25-90 µm.

9. The array substrate according to any one of claims 1-5, further comprising:
a heating electrode (106) between the first substrate (101) and the defining layer (102), wherein the heating electrode (106) is configured to heat the plurality of recesses (103), the heating electrode (106) comprises a plurality of sub-parts separated from each other;
a conductive layer (107) between the first substrate (101) and the heating electrode (106) and electrically connected to the heating electrode (106);
wherein an orthographic projection of at least a portion of the conductive layer (107) on the first substrate (101) falls on a periphery of an orthographic projection of the heating electrode (106) on the first substrate (101), and the conductive layer (107) at least partially surrounds the heating electrode (106).

10. The array substrate according to claim 1, wherein a ratio of an area of the first hydrophobic layer (109) to an area of the hydrophilic layer (108) is between 0.01 and 2.00.

11. A microfluidic device (500), **characterized in that**, the microfluidic device (500) comprises the array substrate according to any one of claims 1-10, a counter substrate (2000) for assembling with the array substrate, and a space between the array substrate and the counter substrate (2000),
wherein the counter substrate (2000) comprises:
a second substrate (201); and
a second hydrophobic layer (202) on a side of the second substrate (201) close to the first substrate (101),
wherein the counter substrate (2000) comprises at least one through hole penetrating the second substrate (201) and the second hydrophobic layer (202), and
wherein the second hydrophobic layer (202) comprises a light-absorbing material, and the light-absorbing material comprises at least one of TiO₂ and TiON.

12. A microfluidic system (600), **characterized in that**, the microfluidic system (600) comprises a control device (620) and the microfluidic device (500) according to claim 11,
wherein the control device (620) is electrically connected to the microfluidic device (500), and is configured to control the temperature of the microfluidic device (500).

13. A fluorescence detection method (700), **characterized in that**, the fluorescence detection method (700) comprises:
containing a reagent to be tested in the plurality of recesses (103) of the microfluidic device (500) according to claim 11;
making a light of a first wavelength (301) emitted by a light source irradiate the plurality of recesses (103) through the plurality of openings (105) defined by the shielding layer (104); and
detecting a light of a second wavelength (302) emitted by the reagent to be tested.

## Patentansprüche

1. Arraysubstrat, umfassend eine Vielzahl von Aussparungen (103), die in einem Array angeordnet ist, wobei sich das Arraysubstrat in einer Ebene befindet und ein Verhältnis einer Fläche einer orthographischen Projektion der Vielzahl von Aussparungen (103) auf die Ebene zu einer Fläche einer orthographischen Projektion des Arraysubstrats auf die Ebene zwischen 0,05 und 0,60 liegt,
wobei das Arraysubstrat ferner umfasst:
ein erstes Substrat (101);
eine Definitionsschicht (102) auf dem ersten Substrat (101) und die die Vielzahl von Aussparungen (103) definiert;
eine Abschirmschicht (104), die eine Vielzahl von Öffnungen (105) definiert, wobei eine orthographische Projektion jeder der Vielzahl von Öffnungen (105) auf dem ersten Substrat (101) mindestens teilweise eine orthographische Projektion einer jeweiligen der Vielzahl von Aussparungen (103) auf dem ersten Substrat (101) überlappt, und eine orthographische Projektion der Abschirmschicht (104) auf dem ersten Substrat (101) mindestens teilweise eine orthographische Projektion der Definitionsschicht (102) auf dem ersten Substrat (101) überlappt;
eine hydrophile Schicht (108); und
eine erste hydrophobe Schicht (109),
wobei die hydrophile Schicht (108) eine Seitenwand und einen Boden jeder der Vielzahl von Aussparungen (103) sowie eine Oberfläche der Definitionsschicht (102) bedeckt, die von dem ersten Substrat (101) entfernt ist, die erste hydrophobe Schicht (109) sich auf einer Seite der Definitionsschicht (102) befindet, die von dem ersten Substrat (101) entfernt ist, und weiter von dem ersten Substrat (101) als die hydrophile Schicht (108) entfernt ist;
**dadurch gekennzeichnet, dass**
die erste hydrophobe Schicht (109) eine Vielzahl von Löchern (114) definiert, wobei die Vielzahl von Löchern (114), die Vielzahl von Aussparungen (103) und die Vielzahl von Öffnungen (105) nacheinander einander entsprechen;
sowohl eine erste orthographische Projektion jedes der Vielzahl von Löchern (114) auf dem ersten Substrat (101) als auch eine dritte orthographische Projektion einer Öffnung (105), die dem Loch (114) auf dem ersten Substrat (101) entspricht, innerhalb einer zweiten orthographischen Projektion einer Aussparung (103) liegen, die dem Loch (114) auf dem ersten Substrat (101) entspricht, und die erste orthographische Projektion, die zweite orthographische Projektion und die dritte orthographische Projektion konzentrische Ringe ausbilden; und
die erste orthographische Projektion zwischen der zweiten orthographischen Projektion und der dritten orthographischen Projektion liegt und die dritte orthographische Projektion innerhalb der ersten orthographischen Projektion liegt.

2. Arraysubstrat nach Anspruch 1, wobei die Vielzahl von Aussparungen (103) die Definitionsschicht (102) durchdringt.

3. Arraysubstrat nach Anspruch 1 oder 2, wobei
die Abschirmschicht (104) sich zwischen dem ersten Substrat (101) und der Definitionsschicht (102) befindet, oder
die Abschirmschicht (104) sich auf einer Seite des ersten Substrats (101) befindet, die von der Definitionsschicht (102) entfernt ist.

4. Arraysubstrat nach Anspruch 1 oder 2,
wobei die Abschirmschicht (104) einen ersten Abschnitt (104a) umfasst, der erste Abschnitt (104a) sich auf einer Seite der Definitionsschicht (102) befindet, die von dem ersten Substrat (101) entfernt ist, und der erste Abschnitt (104a) an Seitenoberflächen der Definitionsschicht (102) und einer Oberfläche der Definitionsschicht (102) angebracht ist, die von dem ersten Substrat (101) entfernt ist; und
wobei der erste Abschnitt (104a) die Vielzahl von Öffnungen (105) definiert.

5. Arraysubstrat nach Anspruch 4,
wobei die Abschirmschicht (104) ferner einen zweiten Abschnitt (104b) umfasst, wobei der zweite Abschnitt (104b) an einer Oberfläche der Definitionsschicht (102) nahe dem ersten Substrat (101) angebracht ist, um die Definitionsschicht (102) zusammen mit dem ersten Abschnitt (104a) zu umgeben;
wobei der zweite Abschnitt (104b) die Vielzahl von Öffnungen (105) definiert; und
wobei eine orthographische Projektion der Vielzahl von Öffnungen (105), die durch den ersten Abschnitt (104a) der Abschirmschicht (104) auf dem ersten Substrat (101) definiert ist, und eine orthographische Projektion der Vielzahl von Öffnungen (105), die durch den zweiten Abschnitt (104b) der Abschirmschicht (104) auf dem ersten Substrat (101) definiert ist, einander vollständig überlappen.

6. Arraysubstrat nach einem der Ansprüche 1 bis 2 und 4 bis 5, wobei eine Oberfläche der Definitionsschicht (102) nahe dem ersten Substrat (101) und/oder eine Oberfläche der Definitionsschicht (102), die von dem ersten Substrat (101) entfernt ist, die Abschirmschicht (104) ausbilden.

7. Arraysubstrat nach einem der Ansprüche 1 bis 5,
wobei die Vielzahl von Aussparungen (103) einer Vielzahl von Öffnungen (105) nacheinander entspricht und eine orthographische Projektion jeder der Vielzahl von Öffnungen (105) auf dem ersten Substrat (101) innerhalb einer orthographischen Projektion einer Aussparung (103) liegt, die der Öffnung (105) auf dem ersten Substrat (101) entspricht.

8. Arraysubstrat nach einem der Ansprüche 1 bis 5, wobei sowohl eine Form einer orthographischen Projektion jeder der Vielzahl von Aussparungen (103) als auch eine Form einer orthographischen Projektion jeder der Vielzahl von Öffnungen (105) auf dem ersten Substrat (101) einen Kreis oder ein regelmäßiges Vieleck umfassen,
wobei die Vielzahl von Aussparungen (103) der Vielzahl von Öffnungen (105) nacheinander entspricht und eine orthographische Projektion jeder der Vielzahl von Öffnungen (105) auf dem ersten Substrat (101) innerhalb einer orthographischen Projektion einer Aussparung (103) liegt, die der Öffnung (105) auf dem ersten Substrat (101) entspricht; und
wobei sowohl eine Form einer orthographischen Projektion jeder Öffnung (105) als auch eine Form einer orthographischen Projektion einer Aussparung (103), die der Öffnung (105) entspricht, auf dem ersten Substrat (101) kreisförmig sind, ein Durchmesser jeder Öffnung (105) in einem Bereich von 20-80 µm liegt und ein Durchmesser der Aussparung (103), die der Öffnung (105) entspricht, in einem Bereich von 25-90 µm liegt; oder eine Form einer orthographischen Projektion jeder Öffnung (105) auf dem ersten Substrat (101) ein erstes regelmäßiges Vieleck ist, und eine Form einer orthographischen Projektion der Aussparung (103), die der Öffnung (105) auf dem ersten Substrat (101) entspricht, ein zweites regelmäßiges Vieleck ist, ein Durchmesser eines Inkreises des ersten regelmäßigen Vielecks in einem Bereich von 20-80 µm liegt, und ein Durchmesser eines Inkreises des zweiten regelmäßigen Vielecks in einem Bereich von 25-90 µm liegt.

9. Verfahren nach einem der Ansprüche 1 bis 5, ferner umfassend:
eine Heizelektrode (106) zwischen dem ersten Substrat (101) und der Definitionsschicht (102), wobei die Heizelektrode (106) konfiguriert ist, um die Vielzahl von Aussparungen (103) zu erhitzen, wobei die Heizelektrode (106) eine Vielzahl von voneinander getrennten Unterteilen umfasst;
eine leitfähige Schicht (107) zwischen dem ersten Substrat (101) und der Heizelektrode (106), die mit der Heizelektrode (106) elektrisch verbunden ist;
wobei eine orthographische Projektion mindestens eines Abschnitts der leitfähigen Schicht (107) auf dem ersten Substrat (101) auf einen Umfang einer orthographischen Projektion der Heizelektrode (106) auf dem ersten Substrat (101) fällt und die leitfähige Schicht (107) die Heizelektrode (106) mindestens teilweise umgibt.

10. Arraysubstrat nach Anspruch 1, wobei ein Verhältnis einer Fläche der ersten hydrophoben Schicht (109) zu einer Fläche der hydrophilen Schicht (108) zwischen 0,01 und 2,00 liegt.

11. Mikrofluidische Vorrichtung (500), **dadurch gekennzeichnet, dass** die mikrofluidische Vorrichtung (500) das Arraysubstrat nach einem der Ansprüche 1 bis 10, ein Gegensubstrat (2000) zum Zusammenbauen mit dem Arraysubstrat und einen Raum zwischen dem Arraysubstrat und dem Gegensubstrat (2000) umfasst,
wobei das Gegensubstrat (2000) umfasst:
ein zweites Substrat (201); und
eine zweite hydrophobe Schicht (202) auf einer Seite des zweiten Substrats (201) nahe dem ersten Substrat (101),
wobei das Gegensubstrat (2000) mindestens ein Durchgangsloch umfasst, das das zweite Substrat (201) und die zweite hydrophobe Schicht (202) durchdringt, und
wobei die zweite hydrophobe Schicht (202) ein lichtabsorbierendes Material umfasst und das lichtabsorbierende Material mindestens eines von TiO₂ und TiON umfasst.

12. Mikrofluidisches System (600), **dadurch gekennzeichnet, dass** das mikrofluidische System (600) eine Steuervorrichtung (620) und die mikrofluidische Vorrichtung (500) nach Anspruch 11 umfasst,
wobei die Steuervorrichtung (620) mit der mikrofluidischen Vorrichtung (500) elektrisch verbunden ist und konfiguriert ist, um die Temperatur der mikrofluidischen Vorrichtung (500) zu steuern.

13. Fluoreszenzdetektionsverfahren (700), **dadurch gekennzeichnet, dass** das Fluoreszenzdetektionsverfahren (700) umfasst:
Enthalten eines zu testenden Reagenzes in der Vielzahl von Aussparungen (103) der mikrofluidischen Vorrichtung (500) nach Anspruch 11;
Veranlassen eines Lichts einer ersten Wellenlänge (301), die durch einer Lichtquelle emittiert wird, die Vielzahl von Aussparungen (103) über die Vielzahl von Öffnungen (105), die durch die Abschirmschicht (104) definiert ist, zu bestrahlen; und
Detektieren eines Lichts einer zweiten Wellenlänge (302), das durch das zu testende Reagenz emittiert wird.

## Revendications

1. Substrat en réseau, comprenant une pluralité d'évidements (103) agencés en réseau, dans lequel le substrat en réseau est dans un plan, et un rapport entre une aire d'une projection orthographique de la pluralité d'évidements (103) sur le plan et une aire d'une projection orthographique du substrat en réseau sur le plan est compris entre 0,05 et 0,60,
dans lequel le substrat en réseau comprend en outre :
un premier substrat (101) ;
une couche de définition (102) sur le premier substrat (101) et définissant la pluralité d'évidements (103) ;
une couche de protection (104) définissant une pluralité d'ouvertures (105), une projection orthographique de chacune de la pluralité d'ouvertures (105) sur le premier substrat (101) chevauchant au moins partiellement une projection orthographique de l'un respectif de la pluralité d'évidements (103) sur le premier substrat (101), et une projection orthographique de la couche de protection (104) sur le premier substrat (101) chevauchant au moins partiellement une projection orthographique de la couche de définition (102) sur le premier substrat (101) ;
une couche hydrophile (108) ; et
une première couche hydrophobe (109),
dans lequel la couche hydrophile (108) recouvre une paroi latérale et un fond de chacun de la pluralité d'évidements (103) ainsi qu'une surface de la couche de définition (102) éloignée du premier substrat (101), la première couche hydrophobe (109) se trouve sur un côté de la couche de définition (102) éloignée du premier substrat (101) et plus éloignée du premier substrat (101) que la couche hydrophile (108) ;
**caractérisé en ce que,**
la première couche hydrophobe (109) définit une pluralité de trous (114), la pluralité de trous (114), la pluralité d'évidements (103) et la pluralité d'ouvertures (105) correspondent l'une à l'autre ;
une première projection orthographique de chacun de la pluralité de trous (114) sur le premier substrat (101) et une troisième projection orthographique d'une ouverture (105) correspondant au trou (114) sur le premier substrat (101) se trouvent toutes deux à l'intérieur d'une deuxième projection orthographique d'un évidement (103) correspondant au trou (114) sur le premier substrat (101), et la première projection orthographique, la deuxième projection orthographique et la troisième projection orthographique forment des anneaux concentriques ; et
la première projection orthographique se trouve entre la deuxième projection orthographique et la troisième projection orthographique, et la troisième projection orthographique se trouve à l'intérieur de la première projection orthographique.

2. Substrat en réseau selon la revendication 1, dans lequel la pluralité d'évidements (103) pénètre dans la couche de définition (102).

3. Substrat en réseau selon la revendication 1 ou 2, dans lequel
la couche de protection (104) se trouve entre le premier substrat (101) et la couche de définition (102), ou
la couche de protection (104) se trouve sur un côté du premier substrat (101) éloignée de la couche de définition (102).

4. Substrat en réseau selon la revendication 1 ou 2,
dans lequel la couche de protection (104) comprend une première partie (104a), la première partie (104a) se trouve sur un côté de la couche de définition (102) éloignée du premier substrat (101), et la première partie (104a) est fixée à des surfaces latérales de la couche de définition (102) et à une surface de la couche de définition (102) éloignée du premier substrat (101) ; et
dans lequel la première partie (104a) définit la pluralité d'ouvertures (105).

5. Substrat en réseau selon la revendication 4,
dans lequel la couche de protection (104) comprend en outre une seconde partie (104b), la seconde partie (104b) est fixée à une surface de la couche de définition (102) proche du premier substrat (101) pour entourer la couche de définition (102) en même temps que la première partie (104a) ;
dans lequel la seconde partie (104b) définit la pluralité d'ouvertures (105) ; et
dans lequel une projection orthographique de la pluralité d'ouvertures (105) définie par la première partie (104a) de la couche de protection (104) sur le premier substrat (101) et une projection orthographique de la pluralité d'ouvertures (105) définie par la seconde partie (104b) de la couche de protection (104) sur le premier substrat (101) se chevauchent complètement.

6. Substrat en réseau selon l'une quelconque des revendications 1 à 2 et 4 à 5, dans lequel une surface de la couche de définition (102) proche du premier substrat (101) et/ou une surface de la couche de définition (102) éloignée du premier substrat (101) constituent la couche de protection (104).

7. Substrat en réseau selon l'une quelconque des revendications 1 à 5,
dans lequel la pluralité d'évidements (103) correspond à la pluralité d'ouvertures (105) une par une, et une projection orthographique de chacune de la pluralité d'ouvertures (105) sur le premier substrat (101) se trouve à l'intérieur d'une projection orthographique d'un évidement (103) correspondant à l'ouverture (105) sur le premier substrat (101).

8. Substrat en réseau selon l'une quelconque des revendications 1 à 5, dans lequel une forme d'une projection orthographique de chacun de la pluralité d'évidements (103) et une forme d'une projection orthographique de chacune de la pluralité d'ouvertures (105) sur le premier substrat (101) comprennent toutes deux un cercle ou un polygone régulier,
dans lequel la pluralité d'évidements (103) correspond à la pluralité d'ouvertures (105) une par une, et une projection orthographique de chacune de la pluralité d'ouvertures (105) sur le premier substrat (101) se trouve à l'intérieur d'une projection orthographique d'un évidement (103) correspondant à l'ouverture (105) sur le premier substrat (101) ; et
dans lequel une forme d'une projection orthographique de chaque ouverture (105) et une forme d'une projection orthographique d'un évidement (103) correspondant à l'ouverture (105) sur le premier substrat (101) sont toutes deux circulaires, un diamètre de chaque ouverture (105) se situe dans une plage comprise entre 20 et 80 µm, et un diamètre de l'évidement (103) correspondant à l'ouverture (105) se situe dans une plage comprise entre 25 et 90 µm ; ou une forme d'une projection orthographique de chaque ouverture (105) sur le premier substrat (101) est un premier polygone régulier, et une forme d'une projection orthographique de l'évidement (103) correspondant à l'ouverture (105) sur le premier substrat (101) est un second polygone régulier, un diamètre d'un cercle inscrit du premier polygone régulier se situe dans une plage comprise entre 20 et 80 µm, et un diamètre d'un cercle inscrit du second polygone régulier se situe dans une plage comprise entre 25 et 90 µm.

9. Substrat en réseau selon l'une quelconque des revendications 1 à 5, comprenant en outre :
une électrode chauffante (106) entre le premier substrat (101) et la couche de définition (102), dans lequel l'électrode chauffante (106) est configurée pour chauffer la pluralité d'évidements (103), l'électrode chauffante (106) comprend une pluralité de sous-parties séparées les unes des autres ;
une couche conductrice (107) entre le premier substrat (101) et l'électrode chauffante (106) et connectée électriquement à l'électrode chauffante (106) ;
dans lequel une projection orthographique d'au moins une partie de la couche conductrice (107) sur le premier substrat (101) se situe sur une périphérie d'une projection orthographique de l'électrode chauffante (106) sur le premier substrat (101), et la couche conductrice (107) entoure au moins partiellement l'électrode chauffante (106).

10. Substrat en réseau selon la revendication 1, dans lequel un rapport entre une aire de la première couche hydrophobe (109) et une aire de la couche hydrophile (108) est compris entre 0,01 et 2,00.

11. Dispositif microfluidique (500), **caractérisé en ce que** le dispositif microfluidique (500) comprend le substrat en réseau selon l'une quelconque des revendications 1 à 10, un contre-substrat (2000) permettant l'assemblage avec le substrat en réseau, et un espace entre le substrat en réseau et le contre-substrat (2000),
dans lequel le contre-substrat (2000) comprend :
un second substrat (201) ; et
une seconde couche hydrophobe (202) sur un côté du second substrat (201) proche du premier substrat (101),
dans lequel le contre-substrat (2000) comprend au moins un trou traversant le second substrat (201) et la seconde couche hydrophobe (202), et
dans lequel la seconde couche hydrophobe (202) comprend un matériau absorbant la lumière, et le matériau absorbant la lumière comprend au moins l'un parmi le TiO₂ et le TiON.

12. Système microfluidique (600), **caractérisé en ce que** le système microfluidique (600) comprend un dispositif de commande (620) et le dispositif microfluidique (500) selon la revendication 11,
dans lequel le dispositif de commande (620) est connecté électriquement au dispositif microfluidique (500) et est configuré pour commander la température du dispositif microfluidique (500).

13. Procédé de détection de fluorescence (700), **caractérisé en ce que** le procédé de détection de fluorescence (700) comprend :
le fait de contenir un réactif à tester dans la pluralité d'évidements (103) du dispositif microfluidique (500) selon la revendication 11 ;
le fait de faire en sorte qu'une lumière d'une première longueur d'onde (301) émise par une source de lumière rayonne la pluralité d'évidements (103) à travers la pluralité d'ouvertures (105) définie par la couche de protection (104) ; et
la détection d'une lumière d'une seconde longueur d'onde (302) émise par le réactif à tester.
